# EUROPÄISCHE PATENTSCHRIFT

(11) **EP 0 668 294 B1**
(45) Veröffentlichungstag und Bekanntmachung des Hinweises auf die Patenterteilung: **18.08.1999**
(21) Anmeldenummer: 95810079.4
(22) Anmeldetag: 07.02.1995
(51) Int. Cl.: C08B 37/16, C07H 13/12, C08F 20/36, G02B 1/04, C07H 15/04, A61K 47/32

(54) **Ungesättigte Kohlenhydratderivate, Polymere davon und deren Verwendung**
Unsaturated carbohydrate derivatives, polymers therefrom and their application
Dérivés insaturés d'hydrates de carbone, leurs polymères et leurs utilisations

(30) Priorität: 15.02.1994 EP 94810084
(43) Veröffentlichungstag der Anmeldung: 23.08.1995
(73) Patentinhaber: Novartis AG, 4058 Basel (CH); Novartis-Erfindungen Verwaltungsgesellschaft m.b.H., 1235 Wien (AT)
(72) Erfinder: Bachmann, Frank, Dr.,, D-79106 Freiburg (DE); Lohmann, Dieter, Dr.,, CH-4142 Münchenstein (CH); Chabrecek, Peter, Dr.,, CH-4055 Basel (CH)

(56) Entgegenhaltungen:
- EP-A- 0 331 633
- EP-A- 0 513 358
- EP-A- 0 530 140
- WO-A-92/13894
- DE-A- 3 904 246
- CHEMICAL ABSTRACTS, vol. 110, no. 12, 20.März 1989 Columbus, Ohio, US; abstract no. 97444y, YAMAKADO NAGAHIKO ET AL. 'Polymerizable unsaturated cellulose derivatives' Seite 116; & DATABASE WPI Week 8836, Derwent Publications Ltd., London, GB; AN 88-252800 & JP-A-63 182 302 (NATOKO PAINT KK) 27 Juli 1988 & PATENT ABSTRACTS OF JAPAN Bd. 12, Nr. 462 (C-549) & JP-A-63 182 302 (NATOKO PAINT KK) 27 Juli 1988
- MAKROMOL. CHEM., RAPID COMMUN., Bd. 10, Seite 629 J. KLEIN ET AL. 'Synthesis and characterization of new poly(vinylsaccharide)s of the urea type.'
- DATABASE WPI Week 8323 Derwent Publications Ltd., London, GB; AN 55552K & SU-A-862 567 (AS LATV WOOD CHEM) , 23.Januar 1983
- Bulletin Soc. chim. belges, Band 99(1990) nr.11-12, seiten 99-110
- DATABASE WPI Week 9145, Derwent Publications Ltd., London, GB; AN 91-329913 & JP-A-3 221 504 (TOPPAN PRINTING CO LTD) 30 September 1991

## Beschreibung

Die vorliegende Erfindung betrifft polymerisierbare Derivate von Kohlenhydraten, enthaltend einen Kohlenhydrat-Rest, gegebenenfalls einen Spacer und eine radikalisch polymerisierbare Kohlenwasserstoffgruppe, Homopolymere, Copolymere, Blockcopolymere, Pfropfcopolymere sowie polymere Netzwerke daraus, Kapseln, Fasern, Filme und Ueberzüge, die wasserbindende und biokompatible Eigenschaften aufweisen, oder Formkörper, z.B. Kontaktlinsen oder biomedizinische Artikel aus den genannten Polymeren, sowie Verfahren zur Herstellung der genannten Polymere und Gegenstände.

Die Einführung von polymerisierbaren Gruppen in Kohlenhydrate wie zum Beispiel Cyclodextrine (CD) ist wegen deren Eigenschaften, besonders der hohen Hydrophilie, des spezifischen Komplexbildungsverhaltens und der Biokompatibilität erwünscht. Acrylhaltige, methacrylhaltige und cinnamoylhaltige Cyclodextrine und Polymere daraus sind zum Beispiel von A.P. Croft et al. in Tetrahedron, Vol. 39, 1425 (1983) beschrieben worden. Die polymerisierbaren Gruppen sind regiospezifisch in den 2- oder 3-Stellungen gebunden. Sie werden durch die Umsetzung von entsprechenden aktivierten Estern, nämlich Carbonsäurenitrophenylestern mit einem Cyclodextrin erhalten. Das entstehende Nitrophenol kann im allgemeinen nur sehr schwer vollständig entfernt werden, da Cyclodextrine mit diesen organischen Verbindungen Einschlussverbindungen bilden. Wegen der physiologischen Bedenklichkeit von Nitrophenolen, deren polymerisationsinhibierender Wirkung und auch der sehr aufwendigen Reinigung können Polymere aus solchen polymerisierbaren Cyclodextrinen nur bedingt verwendet werden.

Infolge der breitgefächerten Anwendungsmöglichkeiten, speziell auf dem pharmazeutischen und anverwandten Sektor, sind Cyclodextrinderivate, die polymerisierbare Gruppen enthalten, breit untersucht worden. So sind beispielsweise in WO 90/02141, WO 91/13100 oder WO 92/09637 solche ungesättigten Cyclodextrin-Derivate beschrieben. Es werden dort auch Polymere aus den genannten ungesättigten Cyclodextrin-Monomeren hergestellt. Solche Polymere enthalten dann Cyclodextrin in immobilisierter Form. Die Herstellung der genannten ungesättigten Cyclodextrinderivate wird immer unter Verwendung von Schutzgruppentechniken und beispielsweise nachfolgender Behandlung mit (Meth)acrylsäurechlorid ausgeführt. In WO 92/09637 wird ein CD beispielsweise mit der sterisch anspruchsvollen tert.-Butyl-diphenylsilylgruppe in einem ersten Schritt an der primären Hydroxygruppe geschützt, dann werden die restlichen OH-Gruppen verethert, die Silylschutzgruppe wird abgespalten und die polymerisierbare Gruppe, (Meth)acrylsäurechlorid, addiert. Das Resultat ist meist unbefriedigend. Aufwendige Synthese- und Reinigungsschritte sind die Regel.

In WO 91/17255 wird eine mit Enzymen katalysierte Herstellung von Polymeren aus Zuckern und Dicarbonsäureestern über eine regioselektive Diacylierung beschrieben, wobei die Zuckerreste als Comonomere im Polymerbackbone gebunden sind und dadurch die typischen Eigenschaften weitgehend verloren gehen.

In Chem. Letters 1990, 1733, wird die Synthese anomerer Glykoside ohne Verwendung von Schutzgruppen beschrieben. Glucosyloxyethylmethacrylat wird z.B. hergestellt aus Methyl-α-D-glucopyranosid und 2-Hydroxyethylmethacrylat (HEMA), wobei ein Katalysator (Phosphormolybdänsäure) und ein Polymerisationsinhibitor (Dinitrochlorbenzol) zugesetzt werden. Es handelt sich hier jedoch um eine Glykosilierungsreaktion.

In Bull. Soc. Chim. Belg. 99, 919(1990), werden ungesättigte Kohlenhydrate durch Umsatz von D-Glucamin oder D-Glucosamin mit 2-Isocyanatoethylmethacrylat (IEM) hergestellt. Die Selektivität basiert hier auf dem unterschiedlichen Reaktionsverhalten von IEM gegenüber den vorhandenen Funktionalitäten, nämlich OH- versus NH₂-Gruppen.

EP-A-331'633 beschreibt hydrophile Monomere, die im wesentlichen aus einer hydrophilen Gruppe und einer polymerisierbaren Seitenkette bestehen. Die hydrophile Gruppe kann auch ein Mono-, Di- oder Polysaccharid sein, wobei das Ausmass der Seitenkettenverknüpfung von eins bis zur vollen Valenz des Saccharides betragen kann, und die polymerisierbare Seitenkette ferner an einer beliebigen Position eingebaut ist.

J. Klein et al. beschreiben in Makromol. Chem., Rapid Commun. 10, 629 (1989) die Synthese von neuen Poly-vinylsacchariden, wobei typischerweise ein 1-Amino-1-desoxysaccarid mit einer stöchiometrischen Menge 2-lsocyanatoethylmethacrylat (IEM) umgesetzt wird. Die Selektivität der Reaktion beruht im wesentlichen in der erhöhten Reaktivität einer Amino- im Vergleich zu einer Hydroxygruppe.

JP 3-21504 beschreibt ein Verfahren zur Herstellung von Cyclodextrin-haltigen Membranen, die typischerweise aus einem Reaktionsprodukt von Polyallylamin mit 6-Jodo-cyclodextrin bestehen.

Es wurde nun überraschend gefunden, dass man völlig ungeschützte Kohlenhydratderivate in einfacher und selektiver Reaktion mit ungesättigten Verbindungen, insbesondere Isocyanaten, zu monosubstituierten Derivaten umsetzen kann. Man isoliert als Resultat einer solchen Umsetzung neue ungesättigte Kohlenhydratderivate, die an einer einzelnen Hydroxygruppe modifiziert sind. Aufwendige Schutzgruppen- und Deblockierungstechniken wie beim Stand der Technik entfallen. Die so erhaltenen ungesättigten Kohlenhydratderivate lassen sich leicht in Polymere überführen, die einen hohen Kohlenhydratanteil enthalten. Homo-, Co- und Blockcopolymere sowie Pfropfcopolymere, linear, verzweigt und vernetzt, sind durch radikalische Polymerisation oder Photopolymerisation zugänglich.

Ein Gegenstand der vorliegenden Erfindung ist somit gerichtet auf eine Verbindung der Formel (I),

R¹-(COO-Alk)ₘ-(OCONH-R)ₙ-(NHCO)ₚ-Y-Z (I)

worin R¹ eine radikalisch polymerisierbare Kohlenwasserstoffgruppe bedeutet; m, n und p die Werte 0 oder 1 haben; Alk Alkylen mit bis zu 10 C-Atomen bedeutet; R ein Diradikal mit bis zu 20 C-Atomen eines organischen Diisocyanates darstellt; Z für einen um eine einzelne primäre Hydroxylgruppe verminderten einwertigen Rest eines Mono-, Di- oder Trisaccharides, eines Oligosaccharides oder eines Cyclodextrins (CD) steht; und Y -O- oder -NH- darstellt; unter der Bedingung, dass wenn p null ist, m und n ebenfalls null sind und Y -NH- bedeutet, ferner unter der Bedingung, dass m, n und p null sind, wenn Y-NH- bedeutet.

Bei R¹ handelt es sich beispielsweise um Alkenyl als radikalisch polymerisierbare Gruppe mit vorzugsweise 2 bis 12 C-Atomen. Beispiele für Alkenyl sind Vinyl, Allyl, 1-Propen-2-yl, 1-Buten-2- oder -3- oder -4-yl, 2-Buten-3-yl, die Isomeren von Pentenyl, Hexenyl, Octenyl, Decenyl und Dodecenyl. R¹ enthält bevorzugt 2 bis 12, besonders bevorzugt 2 bis 8 und insbesondere bevorzugt 2 bis 4 C-Atome.

Das Diradikal R steht beispielsweise für Niederalkylen, Arylen, eine gesättigte bivalente cycloaliphatische Gruppe mit 6 bis 10 Kohlenstoffatomen, Alkylenarylen, Arylenalkylen oder Arylenalkylenarylen.

Arylen ist vorzugsweise Phenylen, das unsubstituiert oder durch Niederalkyl oder Niederalkoxy substituiert ist, insbesondere 1,3-Phenylen oder 1,4-Phenylen oder Methyl-1,4-phenylen.

Eine gesättigte bivalente cycloaliphatische Gruppe ist vorzugsweise Cyclohexylen oder Cyclohexylen-niederalkylen, z.B. Cyclohexylenmethylen, die unsubstituiert ist oder substituiert durch eine oder mehrere Niederalkylgruppen, z.B. Methylgruppen, wie beispielsweise Trimethylcyclohexylenmethylen, z.B. der bivalente Isophoronrest.

Der Begriff "nieder" bedeutet im Rahmen dieser Erfindung im Zusammenhang mit Resten und Verbindungen, soweit er nicht abweichend definiert ist, insbesondere Reste oder Verbindungen mit bis zu 7 Kohlenstoffatomen, vorzugsweise mit bis zu 4 Kohlenstoffatomen.

Niederalkyl weist insbesondere bis zu 7 Kohlenstoffatome auf, vorzugsweise bis zu 4 Kohlenstoffatome, und ist z.B. Methyl, Ethyl, Propyl, Butyl oder tert.-Butyl.

Alkylen weist bis zu 10 Kohlenstoffatome auf und kann geradkettig oder verzweigt sein. Geeignete Beispiele umfassen Decylen, Octylen, Hexylen, Pentylen, Butylen, Propylen, Ethylen, Methylen, 2-Propylen, 2-Butylen oder 3-Pentylen. Alkylen ist vorzugsweise Niederalkylen.

Niederalkylen bedeutet Alkylen mit bis zu 7 und besonders bevorzugt mit bis zu 4 Kohlenstoffatomen. Eine besonders bevorzugte Bedeutung von Niederalkylen ist Methylen und Ethylen.

Die Aryleneinheit von Alkylenarylen oder Arylenalkylen ist vorzugsweise Phenylen, unsubstituiert oder durch Niederalkyl oder Niederalkoxy substituiert, die Alkyleneinheit davon ist vorzugsweise Niederalkylen, wie Methylen oder Ethylen, insbesondere Methylen. Vorzugsweise sind derartige Reste daher Phenylenmethylen oder Methylenphenylen.

Niederalkoxy weist insbesondere bis zu 7 Kohlenstoffatome auf, vorzugsweise bis zu 4 Kohlenstoffatome, und ist z.B. Methoxy, Ethoxy, Propoxy, Butoxy oder tert.-Butoxy.

Arylenalkylenarylen ist vorzugsweise Phenylen-niederalkylen-phenylen mit 7 und insbesondere mit bis zu 4 Kohlenstoffatomen in der Alkyleneinheit, z.B. Phenylenethylenphenylen.

Unter einem Monosaccharid wird im Rahmen der vorliegenden Erfindung eine Aldopentose, Aldohexose, Aldotetrose, Ketopentose oder Ketohexose und verstanden. Die genannten Verbindungen können auch als Lactone vorliegen.

Beispiele für eine Aldopentose sind D-Ribose, D-Arabinose, D-Xylose oder D-Lyose; für eine Aldohexose D-Allose, D-Altrose, D-Glucose, D-Mannose, D-Gulose, D-Idose, D-Galaktose, D-Talose, L-Fucose oder L-Rhamnose; für eine Ketopentose D-Ribulose oder D-Xylulose; für eine Tetrose D-Erythrose oder Threose; für eine Ketohexose D-Psicose, D-Fructose, D-Sorbose oder D-Tagatose.

Beispiele für ein Disaccharid sind Trehalose, Maltose, Isomaltose, Cellobiose, Gentiobiose, Saccharose, Lactose, Chitobiose, N,N-Diacetylchitobiose, Palatinose oder Sucrose.

Als Trisaccharid sei beispielhaft Raffinose, Panose oder Maltotriose genannt.

Als Oligosaccharid sei beispielhaft Maltotetraose, Maltohexaose und Chitoheptaose genannt.

Cyclodextrine enthalten 6 bis 8 gleiche Einheiten von α-1,4-Glucose. Einige Beispiele sind α-, β-, γ-Cyclodextrin, Hydroxypropylcyclodextrin sowie verzweigte Cyclodextrine.

Unter einem Anhydrosaccharid wird ein Saccharid verstanden, das durch Abspaltung von einem oder mehreren Molekülen Wasser aus einem entsprechenden Mono-, Di-, Tri- oder Oligosaccharid gebildet wird.

Beispiele für Anhydrosaccharide sind 1,6-Anhydrosaccharide wie z.B. Levoglucosan (1,6-Anhydro-β-D-glucopyranosid). Weitere mögliche Varianten sind die isomeren 1,2-, 1,3-, 1,4- oder 1,5-Anhydrosaccharide. Beispiele für 1,4-Anhydrosaccharide sind Anhydroerythrit und Threit.

Ein bevorzugtes Anhydrosaccharid ist z.B. Levoglucosan (1,6-Anhydro-β-D-glucopyranosid).

Beispiele für Dianhydrosaccharide sind 1,4:3,6-Dianhydro-D-sorbit, 1,4:3,6-Dianhydro-D-mannit oder 1,4:3,6-Dianhydro-L-idit.

Ein bevorzugtes Dianhydromonosaccharid ist z.B. 1,4:3,6-Dianhydro-D-sorbit.

Ein weiterer Gegenstand der vorliegenden Erfindung ist eine Verbindung der Formel (I), dadurch gekennzeichnet, dass Y -O- bedeutet.

Ebenfalls Gegenstand der vorliegenden Erfindung ist eine Verbindung der Formel (I), dadurch gekennzeichnet, dass m=1, p=1 und n=0 ist, Y einmal für -O- und andererseits für -NH- steht.

Weiterhin Gegenstand der vorliegenden Erfindung ist eine Verbindung der Formel (I), dadurch gekennzeichnet, dass m und n gleichzeitig null sind.

In einer bevorzugten Ausführungsform der Formel (I), bedeutet R¹ Alkenyl mit 2 bis 12, insbesondere mit 2 bis 8 und ganz besonders mit 2 bis 4 C-Atomen.

In einer Verbindung der Formel (I) hat Alk die bevorzugte Bedeutung eines Niederalkylens mit bis zu 7, bevorzugt mit bis zu 4 und stark bevorzugt mit bis zu 2 C-Atomen.

Ein weiterer bevorzugter Gegenstand der vorliegenden Erfindung ist eine Verbindung der Formel (I), worin das Diradikal R Niederalkylen, Arylen, eine gesättigte bivalente cycloaliphatische Gruppe mit 6 bis 10 Kohlenstoffatomen, Alkylenarylen, Arylenalkylen oder Arylenalkylenarylen bedeutet.

Ein bevorzugter Gegenstand der vorliegenden Erfindung ist eine Verbindung der Formel (I), worin der Rest Z sich von einem Monosaccharid ableitet, das insbesondere unter einer Aldohexose und Ketohexose und ganz besonders unter 1-Alkylgucosid ausgewählt ist.

Bevorzugt ist weiterhin eine Verbindung der Formel (I), worin der Rest Z sich von einem Disaccharid ableitet, das unter einer α,α-, α,β- und β,β-Trehalose und insbesondere unter einer α,α-Trehalose ausgewählt ist.

Weiter bevorzugt ist ferner eine Verbindung der Formel (I), worin der Rest Z sich von einem Cyclodextrin ableitet, das unter einem α-, β- und γ-Cyclodextrin, insbesondere unter einem α- und β-Cyclodextrin und ganz besonders unter einem α-Cyclodextrin ausgewählt ist.

Stark bevorzugt ist eine Verbindung der Formel (I), worin R¹ Alkenyl mit 2 bis 8 C-Atomen bedeutet; m und p haben den Wert 1 und n ist null; Alk ist ein Niederalkylen mit bis zu 4 C-Atomen; Y steht für -O-; und der Rest Z leitet sich von einem Saccharid ab, das für ein 1-Alkylglucosid, eine α,α-Trehalose oder ein α- Cyclodextrin steht.

Stark bevorzugt ist ferner eine Verbindung der Formel (I), worin R¹ Alkenyl mit 2 bis 8 C-Atomen bedeutet; m, n und p sind 0; Y steht für -NH-; und der Rest Z leitet sich von einem Saccharid ab, das für ein 1-Alkylglucosid, eine α,α-Trehalose oder ein α- Cyclodextrin steht.

Speziell bevorzugt ist ferner eine Verbindung der Formel (I), worin R¹ Alkenyl mit 2 bis 8 C-Atomen bedeutet; m und n den Wert null haben, und p=1 ist; und der Rest Z sich von einem Saccharid ableitet, das für ein 1-Alkylglucosid, eine α,α-Trehalose oder ein α-Cyclodextrin steht.

Stärker bevorzugt ist eine Verbindung der Formel (I), worin R¹ Alkenyl mit 2 bis 4 C-Atomen bedeutet; m=1, p=1 und n=0 ist; und Alk für Niederalkylen mit bis zu 4 C-Atomen steht.

Extra stark bevorzugt ist eine Verbindung der Formel (I), worin R¹ Alkenyl mit 2 bis 4 C-Atomen bedeutet; m=1, p=1 und n=0 ist; Y -O- bedeutet; und Alk für Niederalkylen mit bis zu 4 C-Atomen steht.

Extra stark bevorzugt ist ferner eine Verbindung der Formel (I), worin R¹ Alkenyl mit 2 bis 4 C-Atomen bedeutet; m=1, p=1 und n=0 ist; Y -O- bedeutet; Alk für Niederalkylen mit bis zu 4 C-Atomen steht; und Z für einen um eine primäre Hydroxylgruppe verminderten einwertigen Rest eines Mono-, Di- oder Trisaccharides, eines Oligosaccharides oder eines Cyclodextrins (CD) steht.

Ein weiterer Gegenstand der vorliegenden Erfindung ist ein Verfahren zur Herstellung einer Verbindung der Formel (I) wie in Anspruch 1 definiert, dadurch gekennzeichnet, dass ein Saccharid der Formel (II)

Z-X (II)

worin Z die in Anspruch 1 angegebene Bedeutung hat, und X für eine reaktionsfähige Gruppe steht, mit einem Derivat der Formel (III)

R¹-(COO-Alk)ₘ-(OCONH-R)ₙ-NCO (III)

oder mit einem Derivat der Formel (IV)

R¹-NH₂ (IV)

umgesetzt wird, wobei die Variablen die in Anspruch 1 angegebene Bedeutung haben.

Die reaktionsfähige Gruppe X ist typischerweise eine Hydroxyl- oder Aminogruppe, die mit einem Isocyanat der Formel (III) ein Urethan oder einen Harnstoff ergibt.

Wird ZX mit einer Verbindung der Formel (IV) umgesetzt, dann steht X generell für ein Nukleofug (austretende Gruppe) und ist insbesondere verestertes oder verethertes Hydroxy.

Beispiele für verestertes Hydroxy sind 4-Toluolsulfonyloxy, 4-Bromsulfonyloxy, Methansulfonyloxy oder Trifluormethylsulfonyloxy, ein Halogenid wie Chlorid, Bromid oder Jodid oder Arylcarbonyloxy wie Dinitrobenzoyloxy oder Benzoyloxy.

Die erfindungsgemässen Verbindungen können in An- oder Abwesenheit von einem Lösungsmittel hergestellt werden. Vorteilhafterweise wird ein Lösungsmittel verwendet, das sich weitgehend inert verhält, d.h. an der Reaktion nicht teilnimmt. Geeignete Beispiele hierfür sind Ether wie Tetrahydrofuran (THF), Diethylether, Diethylenglykolmonomethylether oder Dioxan, halogenierte Kohlenwasserstoffe wie Chloroform oder Methylenchlorid, bipolar aprotische Lösungsmittel wie Acetonitril, Aceton, Dimethylformamid (DMF), Hexamethylphosphoramid (HMPA), N-Methylpyrrolidon (NMP) oder Dimethylsulfoxid (DMSO), Alkohole wie Ethanol oder Methanol, ferner Pyridin oder N-Methylmorpholin.

Bei der Herstellung der erfindungsgemässen Verbindungen werden die Reaktanden vorteilhaft in equimolaren Mengen einsetzt. Die Reaktionstemperatur kann zum Beispiel von -30 bis 150°C betragen. Der Bereich von 0°C bis Raumtemperatur ist ein bevorzugter Temperaturbereich. Die Reaktionszeiten liegen im Bereich von etwa 15 Minuten bis zu 7 Tagen, vorzugsweise etwa 12 Stunden. Falls erforderlich wird unter Argon oder Stickstoff als Schutzgas gearbeitet.

Ein weiterer Gegenstand der vorliegenden Erfindung ist ein Polymer enthaltend ein Polymerisationsprodukt von mindestens einer Verbindung der Formel (I) gemäss der vorstehenden Definition und gegebenenfalls von mindestens einem weiteren davon verschiedenartigen vinylischen Comonomer (a), wobei ein Polymer ausgenommen ist, das durch Umsetzung von 6-Iodo-6-desoxy-β-cyclodextrin mit Polyallylamin erhältlich ist.

Die bevorzugte Zusammensetzung eines erfindungsgemässen Polymers ist dadurch gekennzeichnet, dass der Gewichtsanteil bezüglich Gesamtpolymer einer Verbindung der Formel (I) im Bereich von 100 - 0.5 %, insbesondere im Bereich von 80 - 2 % und bevorzugt im Bereich von 70 - 5 % liegt.

Ein bevorzugtes Polymer enthaltend ein Polymerisationsprodukt von mindestens einer Verbindung der Formel (I) ist dadurch gekennzeichnet, dass Comonomer (a) abwesend ist und es sich um ein Homopolymer handelt.

Ein Comonomer (a), das in einem erfindungsgemässen Polymer enthalten ist, kann hydrophil oder hydrophob, oder ein Gemisch der beiden sein. Geeignete Comonomere umfassen insbesondere solche, die üblicherweise bei der Herstellung von Kontaktlinsen und biomedizinischen Materialien verwendet werden.

Unter einem hydrophoben Comonomer (a) werden Monomere verstanden, die als Homopolymer typischerweise Polymere ergeben, die wasserunlöslich sind und weniger als 10 Gewichtsprozent Wasser absorbieren können.

Analog wird unter einem hydrophilen Comonomer (a) ein Monomer verstanden, das als Homopolymer typischerweise ein Polymer ergibt, das wasserlöslich ist oder mindestens 10 Gewichtsprozent Wasser absorbieren kann.

Geeignete hydrophobe Comonomere (a) umfassen, ohne dass diese Aufzählung abschliessend wäre, C₁-C₁₈-Alkyl- und C₃-C₁₈-Cycloalkylacrylate und -methacrylate, C₃-C₁₈-Alkylacrylamide und -methacrylamide, Acrylnitril, Methacrylnitril, Vinyl-C₁-C₁₈-alkanoate, C₂-C₁₈-Alkene, C₂-C₁₈-Haloalkene, Styrol, Niederalkylstyrol, Niederalkylvinylether, C₂-C₁₀-Perfluoralkyl-acrylate und -methacrylate oder entsprechend partiell fluorierte Acrylate und Methacrylate, C₃-C₁₂-Perfluoralkyl-ethyl-thiocarbonylaminoethyl-acrylate und -methacrylate, Acryloxy und Methacryloxy-alkylsiloxane, N-Vinylcarbazol, C₁-C₁₂-Alkylester der Maleinsäure, Fumarsäure, Itaconsäure, Mesaconsäure und dergleichen. Bevorzugt sind z.B. Acrylnitril, C₁-C₄-Alkylester von vinylisch ungesättigten Carbonsäuren mit 3 bis 5 Kohlenstoffatomen oder Vinylester von Carbonsäuren mit bis zu 5 Kohlenstoffatomen.

Beispiele geeigneter hydrophober Comonomere (a) umfassen Methylacrylat, Ethylacrylat, Propylacrylat, Isopropylacrylat, Cyclohexylacrylat, 2-Ethylhexylacrylat, Methylmethacrylat, Ethylmethacrylat, Propylmethacrylat, Butylacrylat, Vinylacetat, Vinylpropionat, Vinylbutyrat, Vinylvalerat, Styrol, Chloropren, Vinylchlorid, Vinylidenchlorid, Acrylnitril, 1-Buten, Butadien, Methacrylnitril, Vinyltoluol, Vinylethylether, Perfluorhexylethylthiocarbonylaminoethylmethacrylat, Isobornylmethacrylat, Trifluorethylmethacrylat, Hexafluorisopropylmethacrylat, Hexafluorbutylmethacrylat, Tris-trimethylsilyloxy-silyl-propylmethacrylat, 3-Methacryloxypropylpentamethyldisiloxan und Bis(methacryloxypropyl)-tetramethyldisiloxan.

Bevorzugte Beispiele hydrophober Comonomere (a) sind Methylmethacrylat und Acrylnitril.

Geeignete hydrophile Comonomere (a) umfassen, ohne dass diese Aufzählung abschliessend wäre, durch Hydroxy substituierte Niederalkylacrylate und -methacrylate, Acrylamid, Methacrylamid, Niederalkylacrylamide und -methacrylamide, ethoxylierte Acrylate und Methacrylate, durch Hydroxy substituierte Niederalkylacrylamide und Methacrylamide, durch Hydroxy substituierte Niederalkylvinylether, Natriumvinylsulfonat, Natriumstyrolsulfonat, 2-Acrylamido-2-methylpropansulfonsäure, N-Vinylpyrrol, N-Vinyl-2-pyrrolidon, 2- und 4-Vinylpyridin, vinylisch ungesättigte Carbonsäuren mit insgesamt 3 bis 5 Kohlenstoffatomen, Aminoniederalkyl- (wobei der Begriff "Amino" auch quaternäres Ammonium umfasst), Mononiederalkylamino-niederalkyl- und Diniederalkylamino-niederalkylacrylate und -methacrylate, Allylalkohol und dergleichen. Bevorzugt sind z.B. N-Vinyl-2-pyrrolidon, Acrylamid, Methacrylamid, durch Hydroxy substituierte Niederalkylacrylate und -methacrylate, durch Hydroxy substituierte Niederalkylacrylamide und -methacrylamide und vinylisch ungesättigte Carbonsäuren mit insgesamt 3 bis 5 Kohlenstoffatomen.

Beispiele geeigneter hydrophiler Comonomere (a) umfassen Hydroxyethylmethacrylat, Hydroxyethylacrylat, Hydroxypropylacrylat, Ammoniumethylmethacrylat-hydrochlorid, Acrylamid, Methacrylamid, N,N-Dimethylacrylamid, Allylalkohol, Vinylpyridin, Glycerinmethacrylat, N-(1,1-Dimethyl-3-oxobutyl)acrylamid, N-Vinyl-2-pyrrolidon, Acrylsäure, Methacrylsäure und dergleichen.

Bevorzugte hydrophile Comonomere (a) sind 2-Hydroxyethylmethacrylat, Acrylamid, N,N-Dimethylacrylamid und N-Vinyl-2-pyrrolidon.

Die erfindungsgemässen Polymere werden auf an sich bekannte Art aus den entsprechenden Monomeren durch eine dem Fachmann geläufige Polymerisationsreaktion aufgebaut. Ueblicherweise wird ein Gemisch von den vorstehend genannten Monomeren unter Zusatz eines Radikalbildners erwärmt. Ein solcher Radikalbildner ist z.B. Azaisobutyronitril (AIBN), Kaliumperoxodisulfat, Dibenzoylperoxid, Wasserstoffperoxid, Natriumpercarbonat oder andere. Werden beispielsweise die genannten Verbindungen erwärmt, so bilden sich unter Homolyse Radikale, die dann beispielsweise eine Polymerisation einleiten können.

Besonders bevorzugt sind Redoxpolymerisationsinitiatoren wie z.B. die Gemische Ammoniumperoxodisulfat / Natriumdisulfit oder Fe²⁺(z.B. FeSO₄)/Wasserstoffperoxid.

Eine Polymerisation kann in An- oder Abwesenheit von einem Lösungsmittel durchgeführt werden. Als Lösungsmittel sind grundsätzlich alle Lösungsmittel geeignet, die die verwendeten Monomere lösen, z.B. Wasser, Alkohole wie Niederalkanole, z.B. Ethanol oder Methanol, ferner Carbonsäureamide, wie Dimethylformamid, dipolar aprotische Lösungsmittel wie Dimethylsulfoxid, ebenso Gemische von geeigneten Lösungsmitteln, wie z.B. Gemische von Wasser mit einem Alkohol, wie z.B. ein Wasser/Ethanol- oder ein Wasser/Methanol-Gemisch.

Ein weiterer Gegenstand der vorliegenden Erfindung sind polymere Netzwerke, bestehend im wesentlichen aus einem Polymer, enthaltend ein Polymerisationsprodukt von mindestens einer Verbindung der Formel (I) und gegebenenfalls von mindestens einem weiteren davon verschiedenartigen vinylischen Comonomer (a), in vernetzter Form.

Ein weiterer Gegenstand der vorliegenden Erfindung sind polymere Netzwerke, bestehend im wesentlichen aus einem Polymer, enthaltend ein Polymerisationsprodukt von mindestens einer Verbindung der Formel (I) in Abwesenheit eines Comonomers (a), in vernetzter Form.

Ein weiterer Gegenstand der vorliegenden Erfindung ist ein Verfahren zur Herstellung von polymeren Netzwerken, das dadurch gekennzeichnet ist, dass man ein erfindungsgemässes Polymer vernetzt; z.B. durch energiereiche oder ionisierende Strahlen, oder durch eine chemische Reaktion wie z.B. durch Umsetzen mit einem Quervernetzer wie z.B. einem Diisocyanat.

Die Vernetzung eines erfindungsgemässen Polymers wird gegebenenfalls unter Zusatz eines vorzugsweise divinylischen Comonomers, wie z.B. Allyl(meth)acrylat, Niederalkylenglykoldi(meth)acrylat, Divinylether, Divinylbenzol, Bisphenol-A-di(meth)acrylat, Methylenbis(meth)acrylamid oder Diallylphthalat, durch radikalische oder vorzugsweise durch Photovernetzung bewerkstelligt.

Bei der Photovernetzung wird geeigneterweise ein Photoinitiator zugesetzt, der eine radikalische Vernetzung initiieren kann. Beispiele hierfür sind dem Fachmann geläufig, speziell können als geeignete Photoinitiatoren Benzoinmethylether, 1-Hydroxycyclohexylphenylketon, Darocur und Irgacure-Typen, bevorzugt Darocur 1173® und Irgacur 2959® genannt werden. Die Vernetzung kann dann durch aktinische Strahlung, wie z.B. UV-Licht geeigneter Wellenlänge ausgelöst werden.

Auch geeignet sind solche Photoinitiatoren, die bereits vor dem Vernetzungsschritt im Polymer eingebaut werden. Beispiele speziell geeigneter Photoinitiatoren sind dem Fachmann bekannt und sind insbesondere Derivate von Irgacure 2959®, die beispielsweise mit Methacrylsäure verestert sind. Eine mit z.B. Irgacur 2959® veresterte Methacrylsäure kann nun als spezielles Monomer in ein Polymer eingebaut werden. Ein solches Polymer ist nun geeignet, direkt, ohne Zusatz eines Photoinitiators, vernetzt zu werden.

Eine Vernetzung wird gegebenenfalls in einem Lösungsmittel durchgeführt. Als Lösungsmittel sind grundsätzlich alle Lösungsmittel geeignet, die die Polymere lösen, z.B. Wasser, Alkohole wie Niederalkanole, z.B. Ethanol oder Methanol, ferner Carbonsäureamide, wie Dimethylformamid, oder Dimethylsulfoxid, ebenso Gemische von geeigneten Lösungsmitteln, wie z.B. Gemische von Wasser mit einem Alkohol, wie z.B. ein Wasser/Ethanol- oder ein Wasser/Methanol-Gemisch.

Die erfindungsgemässen Polymeren und polymeren Netzwerke können auf an sich bekannte Weise zu Formkörpern verarbeitet werden, insbesondere zu Kontaktlinsen, beispielsweise indem die Photovernetzung der erfindungsgemässen Polymere in einer geeigneten Kontaktlinsenform erfolgt. Ein weiterer Gegenstand der Erfindung ist daher auf Formkörper gerichtet, die im wesentlichen aus erfindungsgemässen Polymeren oder polymeren Netzwerken bestehen. Weitere Beispiele für erfindungsgemässe Formkörper, neben Kontaktlinsen, sind biomedizinische Artikel oder speziell ophthalmische Formkörper, z.B. künstliche Hornhaut, Intraokularlinsen, Augenverbände, Formkörper, die in der Chirurgie Verwendung finden können, wie Herzklappen, künstliche Arterien oder dergleichen, ferner Filme oder Membranen, z.B. Membranen für Diffusionskontrolle, photostrukturierbare Folien für Informationsspeicherung, oder Photoresistmaterialien, z.B. Membranen oder Formkörper für Aetzresist oder Siebdruckresist.

Eine spezielle Ausführungsform der Erfindung ist auf Kontaktlinsen gerichtet, die ein erfindungsgemässes polymeres Netzwerk umfassen oder im wesentlichen oder vollständig aus einem erfindungsgemässen polymeren Netzwerk bestehen. Derartige Kontaktlinsen weisen eine Palette ungewöhnlicher und äusserst vorteilhafter Eigenschaften auf. Unter diesen Eigenschaften sind beispielsweise ihre ausgezeichnete Verträglichkeit mit der menschlichen Cornea sowie mit der Tränenflüssigkeit zu nennen, die auf einem ausgewogenen Verhältnis von Wassergehalt, Sauerstoffdurchlässigkeit und mechanischen sowie adsorptiven Eigenschaften beruhen. Im übrigen sind die erfindungsgemässen Kontaktlinsen von hoher Formbeständigkeit.

Alle die vorstehend genannten Vorteile gelten naturgemäss nicht nur für Kontaktlinsen, sondern auch für andere erfindungsgemässe Formkörper.

Ein weiterer Gegenstand der vorliegenden Erfindung ist eine Kontaktlinse erhältlich durch Vernetzen eines erfindungsgemässen Polymers.

Eine spezielle Verwendung der erfindungsgemässen Polymere und polymeren Netzwerke ist gerichtet auf Abgabe-Systeme biologischer Wirkstoffe, beispielsweise pharmazeutischer Wirkstoffe (Drug Delivery Systeme). Die erfindungsgemässen Polymere und polymeren Netzwerke besitzen eine Gelstruktur, in welche organische Verbindungen, insbesondere pharmazeutisch aktive organische Verbindungen gegebenenfalls eingelagert werden können. Werden solche Polymere z.B. lokal verabreicht, dann entfalten die pharmazeutischen Wirkstoffe ihre Wirkung erstens durch langsame und kontinuierliche Abgabe, nämlich diffusionskontrolliert, und zweitens lokal begrenzt, da an ein Substrat (Carrier) gebunden.

Ein weiterer Gegenstand der vorliegenden Erfindung ist gerichtet auf die Verwendung der erfindungsgemässen Monomere der Formel (I), oder der daraus hergestellten und vorstehend beschriebenen Polymere, für die Beschichtung von einem Grundmaterial, wie z.B. Glas, Keramik oder Metall, sowie bevorzugt von Polymersubstraten, wie z.B. ophthalmisch verwendbare Produkte wie Kontaktlinsen, Intraokularlinsen oder Augenverbände, sowie von medizinisch verwendbaren Produkten.

Die Verbindungen der Formel (I) und die daraus hergestellten Polymere sind besonders geeignet, um vorgeformte Polymersubstrate, speziell ophthalmisch verwendbare Produkte, wie Kontaktlinsen, mit einem hydrophilen Film zu beschichten.

Unter Polymersubstraten werden daher insbesondere Substrate aus für ophthalmologische Linsen, speziell Kontaktlinsen, typischen Materialien verstanden. Geeignete Polymersubstrate sind beispielsweise RGP-Linsen (Rigid Gas Permeable), z.B. Nefocon A (Ocusil), Pasifocon A (Paraperm-02), Telefocon B (SGP-II), Silafocon A (Polycon-2), Fluorsilfocon (Fluorex-400), Paflufocon A (Fluoroperm-30) oder Silafocon B (Polycon-HDK) verstanden; ebenfalls geeignet sind amorphe Teflonsubstrate oder Kontaktlinsen daraus, beispielsweise solche aus Teflon AF 1600 oder Teflon AF 2400, wobei das erste ein Copolymer von 63-73 Mol% Perfluor-2,2-dimethyl-1,3-dioxol und 37-27 Mol% Tetrafluorethylen und das zweite ein Copolymer von 80-90 Mol% Perfluor-2,2-di-methyl-1,3-dioxol und 20-10 Mol% Tetrafluorethylen ist. Insbesondere sind Polymersubstrate geeignet, die Polysiloxane enthalten.

Die Beschichtung eines genannten Grundmaterials wird in der Regel mit einer dem Fachmann bekannten Methode ausgeführt. Eine erfindungsgemässe Verbindung der Formel (I) oder daraus hergestellte Polymere werden z.B. kovalent mittels reaktiver Gruppen auf einer Körperoberfläche gebunden. Falls das zu beschichtende Material keine geeigneten reaktiven Gruppen an seiner Körperoberfläche aufweist, so behandelt man es beispielsweise zunächst mit einem Plasma. Dabei werden geeignete reaktive Gruppen auf der Oberfläche des besagten Grundmaterials eingebaut. Diese können jetzt z.B. mit einem difunktionellen Radikal derivatisiert werden, das nun seinerseits mit einer erfindungsgemässen Verbindung der Formel (I) eine kovalente Bindung eingehen kann.

Geeignete reaktive Gruppen sind beispielsweise Hydroxy, Amino, Carboxy, Carbonyl, Sulfonyl, Sulfonylchlorid oder Halogene wie Brom oder Jod. Bevorzugte reaktive Gruppen sind Hydroxy und Amino. Die Methode um reaktive Gruppen wie Hydroxy oder Amino via Plasmaoberflächenbehandlung auf eine Körperoberfläche aufzubringen, ist beispielsweise in der PCT Anmeldung WO 89/00220 (Griesser et al.) umfassend beschrieben.

Um erfindungsgemässe Monomere der Formel (I) und die daraus hergestellten Polymere auf eine Körperoberfläche aufpfropfen zu können, muss die Körperoberfläche zuerst, wie vorstehend erwähnt, derivatisiert werden. Dies wird zweckmässig z.B. mit einem difunktionellen Radikal ausgeführt, das dadurch gekennzeichnet ist, dass seine funktionellen Gruppen einerseits mit den z.B. Hydroxy- oder Aminogruppen der Körperoberfläche und andererseits mit den z.B. Hydroxygruppen der Verbindungen der Formel (I) oder den daraus hergestellten Polymeren kovalente Bindungen ausbilden. Die funktionellen Gruppen des difunktionellen Radikals sind bevorzugt Isocyanate und das Radikal ist ausgewählt unter Niederalkylen, Arylen, einer gesättigten bivalenten cycloaliphatischen Gruppe mit 6 bis 10 Kohlenstoffatomen, Alkylenarylen, Arylenalkylen und Arylenalkylenarylen.

Eine weitere Methode um erfindungsgemässe Monomere der Formel (I) oder daraus hergestellte Polymere auf eine Körperoberfläche aufzupfropfen, besteht im wesentlichen darin, dass eine photoreaktive Gruppe an ein erfindungsgemässes Monomer der Formel (I) oder an ein daraus hergestelltes Polymer gebunden wird, das dann beim Bestrahlen mit UV-Licht geeigneter Wellenlänge an eine, z.B. mit Plasmasauerstoff vorbehandelte, Körperoberfläche ankoppelt. Diese Methode ist umfassend beschrieben in US 5 002 582 oder von R.L.W. Smithson et al., Colloids and Surfaces B: Biointerfaces, 1, 349 -355(1993).

Eine dritte Methode um erfindungsgemässe Monomere der Formel (I) oder daraus hergestellte Polymere auf eine Körperoberfläche aufzubringen besteht darin, dass zunächst ein reaktiver Photoinitiator an die Körperoberfläche gebunden wird und dann mit einer Photopfropfpolymerisation Monomere der Formel (I) aufgepfropft werden. Es entstehen spezielle Pfropfpolymerschichten mit einer sogenannten Bürstenstruktur, die auch noch vernetzt oder verzweigt sein können.

Die nachfolgend aufgeführten Beispiele dienen der weiteren Illustration der vorliegenden Erfindung; sie sollen diese jedoch in ihrem Umfang in keiner Weise einschränken.
Temperaturen sind in Grad Celsius angegeben.

### Beispiel 1

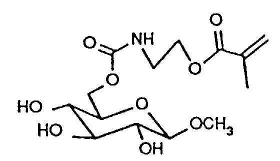

### 6-O-Carbamoyl-methacryloylethyl-methyl-β-D-glucopyranosid

37g (0.19 mol) Methyl-β-D-glucopyranosid werden bei 0°C in 500 ml Pyridin gelöst und mit einem Molequivalent 2-Isocyanatoethylmethacrylat (IEM) tropfenweise versetzt. Man lässt zwei Tage bei 0°C rühren (DC-Kontrolle, Laufmittel Chloroform/MeOH 10:1). Das Pyridin wird bei Raumtemperatur vorsichtig abdestilliert. Der Rückstand wird chromatographiert mit Chloroform/MeOH 10:1. Es verbleiben nach dem Entfernen der Lösungsmittel 39.8 g (60 %) eines farblosen, hygroskopischen Schaumes.
MS(FAB): 348(M - H)⁻, 384(M + Cl)⁻

### Beispiel 2

### 6-O-Carbamoyl-methacryloylethyl-octyl-β-D-glucopyranosid

208 mg (0.71 mmol) Octyl-β-D-glucopyranosid werden in Analogie zu Beispiel 1 mit einem Molequivalent 2-Isocyanatoethylmethacrylat versetzt. Nach Rühren über Nacht wird analog zu Beispiel 1 aufgearbeitet und gereinigt. Es verbleiben 130 mg (41 %) eines farblosen Oels.
MS(FAB): 446(M - H)⁻, 482(M + Cl)⁻

### Beispiel 3

### 6-O-Carbamoyl-methacryloylethyl-methyl-α-D-glucopyranosid

2.0 g (10.3 mmol) Methyl-α-D-glucopyranosid werden in 20 ml Pyridin bei 0°C gelöst. Zu dieser Lösung gibt man ein Molequivalent 2-Isocyanatoethylmethacrylat. Nach 6 Stunden gibt man ein weiteres Equivalent 2-Isocyanatoethylmethacrylat zu und lässt über Nacht rühren. Nach 24 Stunden wird ein drittes Equivalent 2-Isocyanatoethylmethacrylat zugegeben. Nach 36 Stunden wird die Reaktionslösung mit 20 ml Toluol verdünnt und anschliessend eingeengt. Der Rückstand wird über 500 g Kieselgel chromatographisch gereinigt. Die Eluationsmittel sind: Essigsäureethylester (2000 ml), Essigsäureethylester/Acetonitril 9:1 (2000 ml), Essigsäureethylester / Acetonitril 1:1 (1000 ml) und Methanol (1000 ml). Man erhält das α-Analoge von Beispiel 1, die Titelverbindung, als farbloses Pulver.
MS(FAB): 349(M)⁺, 367(M + NH₄)⁺

### Beispiel 4

### Methyl-6-N-(ureido-methacryloylethyl)-6-desoxy-α-D-glucopyranosid

0.276 g (1.2 mmol) Methyl-6-amino-6-desoxy-α-D-glucopyranosid-hydrochlorid werden in 10 ml Pyridin bei 0°C gelöst und mit 50 mg Diazabicyclo[2.2.2]octan versetzt. Man gibt tropfenweise 1 Molequivalent 2-Isocyanatoethylmethacrylat dazu und lässt über Nacht rühren (DC-Kontrolle mit Ammoniak [25 % wässrig], Ether, Isopropanol 5:5:6). Die Reaktionslösung wird dann vorsichtig eingeengt und chromatographisch mit dem Lösungsmittelgemisch CH₃CN / Methanol 9:1 gereinigt. Die Titelverbindung verbleibt als farbloses Oel.
MS(FAB): 347(M - H)⁻, 383(M + Cl)⁻

### Beispiel 5

### 6-O-Carbamoyl-methacryloylethyl-α,α-trehalose

40 g (0.106 mol) α,α-Trehalose werden in 400 ml Pyridin gelöst und unter Rühren bei Raumtemperatur (RT) 18.13 g (0.117 mol, 1.1 Equivalent) 2-Isocyanatoethylmethacrylat langsam zugetropft. Man lässt über Nacht rühren. Die Reaktionslösung wird dann mit 300 ml Toluol versetzt, wobei ein weisser Feststoff ausfällt. Man filtriert und wäscht den Rückstand mit wenig Toluol nach. Man erhält so 39.4 g Rohprodukt, die in 300 ml Wasser gelöst werden. Zu dieser Lösung wird langsam 1.2 Liter Acetonitril gegeben, wobei intermediär eine Emulsion entsteht. Es wird daher ein weiterer Liter Acetonitril zugegeben, wobei ein weisser Feststoff ausfällt. Dieser wird abfiltriert und als Ausgangsmaterial zurückgewonnen (18 g, 45 %). Die klare Lösung wird dann weiter so lange mit Aceton versetzt, bis keine weitere Ausfällung mehr auftritt. Die klarfiltrierte Lösung wird dann eingeengt. Der verbleibende Rückstand wird in Wasser gelöst, mit Essigsäureethylester zweimal gewaschen und erneut eingeengt. Man erhält so die Titelverbindung als farblosen Feststoff.
R_{f} - Wert: 0.59 (CH₃CN / H₂O 8:2)
MS(FAB): 496(M-H)⁻

| Verbrennungsanalyse: | | | |
|---|---|---|---|
| | C | H | N |
| berechnet | 45.88 % | 6.28 % | 2.82 % |
| gefunden | 45.12 % | 6.29 % | 2.84 % |

### Beispiel 6

### 6-O-Carbamoyl-methacryloylethyl-β,β-trehalose

In einem Kolben wird unter Argon 1.9 g (0.292 mmol) β,β-Trehalose in 19 ml trockenem Pyridin gelöst Zu dieser Lösung wird bei Raumtemperatur 861 µl (5.55 mmol) IEM langsam zugetropft. Nach einer Stunde ist bereits ein deutlicher Umsatz zu erkennen. Man rührt noch weitere 6 Stunden und arbeitet dann auf. Es wird 10 ml Toluol zugegeben, wobei ein Feststoff ausfällt, der in Wasser gelöst und mittels Chromatographie gereinigt wird (300 g Kieselgel, Acetonitril / Wasser 9:1). Es verbleiben 1.0 g (36 %) eines farblosen Feststoffes.
R_{f} - Wert: 0.48 (CH₃CN / H₂O 8:2).

### Beispiel 7

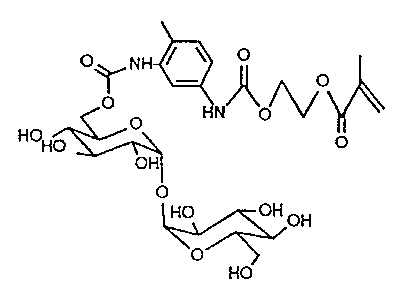

### 6-O-Carbamoyl-{(methacryloylethyl-O-carbamoyl)-2,4-toluoyl}-α,α-trehalose

2 g (5.3 mmol) α,α-Trehalose wird in 100 ml Pyridin bei 0°C gelöst. Dann wird 1 Equivalent 2-Isocyanato-4-N-(carbamoyl-methacryloylethyl)-toluol (hergestellt aus 2,4-Toluylendiisocyanat und 2-Hydroxyethylmethacrylat gemäss US 2 958 704) zugegeben. Nach 24 Stunden wird das Reaktionsgemisch im Hochvakuum eingeengt und der Rückstand chromatographisch gereinigt (Kieselgel, Acetonitril / Wasser 8:2). Die Titelverbindung verbleibt als farbloser Feststoff.
MS(FAB): 645(M -H)⁻, 669(M + Na)⁺, 681(M + Cl)⁻

### Beispiel 8

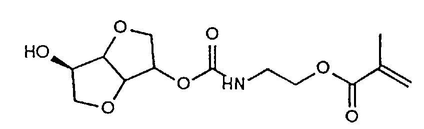

### 2-O-resp.(5-O)-Monocarbamoyl-methacryloylethyl-1,4:3,6-dianhydro-D-sorbit

5 g (34 mmol) 1,4:3,6-Dianhydro-D-sorbit werden in 50 ml Pyridin gelöst und bei 0°C mit 1 Equivalent 2-Isocyanatoethylmethacrylat versetzt. Man lässt auf RT erwärmen und rührt über Nacht weiter. Das Reaktionsgemisch wird dann eingeengt und chromatographisch gereinigt. (Kieselgel, Chloroform / Methanol 10:1) Die Titelverbindungen werden als farblose Oele erhalten.

### Beispiel 9

### 6-O-Carbamoyl-methacryloylethyl-α,β-maltotriose

5 g (9.9 mmol) Maltotriose in 100 ml Pyridin werden bei RT mit 1 Equivalent 2-Isocyanatoethylmethacrylat versetzt. Nach 48 Stunden gibt man 100 ml Toluol zu und engt vorsichtig im Hochvakuum zur Trockene ein. Es verbleibt ein hellgelbes Oel, das chromatographisch gereinigt wird (400 g Kieselgel, Acetonitril/Wasser zuerst 9:1, dann 8:2). Man erhält 1.3 g (20 %) Monoacrylat als farbloses Oel.
R_{f} - Wert: 0.35 (CH₃CN / H₂O 8:2)
MS(FAB): 658(M - H)⁻, 694(M + Cl)⁻

### Beispiel 10

### 6-O-Carbamoyl-methacryloylethyl-α-cyclodextrin

5 g (5.14 mmol) α-Cyclodextrin (α-CD) werden in 50 ml Pyridin gelöst und bei RT mit 1 Equivalent 2-Isocyanatoethylmethacrylat versetzt. Nach 24 Stunden hat sich eine Suspension gebildet, die filtriert wird. Man erhält so 3.9 g α-CD Ausgangsmaterial zurück. Die klare Lösung wird dann mit 200 ml Toluol versetzt, wobei ein weisser Niederschlag ausfällt. Dieser wird abgesaugt und getrocknet. Dieser getrocknete Niederschlag wird in 10 ml Wasser gelöst und 60 ml Aceton versetzt. Es entsteht erneut ein Niederschlag, der abfiltriert und verworfen wird. Die verbleibende klare Lösung wird eingeengt, in wenig Methanol aufgenommen und dann mit einigen Tropfen Acetonitril versetzt. Es ensteht ein weisser Niederschlag, der abgesaugt und getrocknet wird. Die Titelverbindung wird so ohne chromatographische Reinigung erhalten.

Alternativ lässt sich das Rohprodukt durch chromatographische Reinigung an Kieselgel mit Acetonitril / Wasser 9:1, dann 8:2 und schliesslich mit 7:3, rein darstellen.
R_{f} - Wert: 0.24 (CH₃CN / H₂O 8:2)
MS(FAB): 1126(M - H)⁻, 1162(M + Cl)⁻

### Beispiel 11

### 6-O-Carbamoyl-methacryloylethyl-β-cyclodextrin

1.0 g (0.88 mmol) β-CD werden in 15 ml Pyridin gelöst und bei 0°C mit 2.74 g (1.8 mmol) 2-Isocyanatoethylmethacrylat (IEM) tropfenweise verdünnt. Nach 2 Tagen wird das Reaktionsgemisch mit 100 ml Toluol versetzt und dann im Vakuum vollständig eingeengt. Der Rückstand wird über Kieselgel chromatographiert mit Acetonitril / Wasser 8:2. Nach dem Entfernen des Lösungsmittels verbleibt die Titelverbindung in amorpher Form.
R_{f} - Wert: 0.16 (CH₃CN / H₂O 8:2)
MS(FAB): 1288(M - H)⁻

### Beispiel 12

### 6-O-Monoallylcarbamoyl-β-cyclodextrin

2.0 g (1.76 mmol) β-CD werden in 20 ml Pyridin gelöst und bei 0°C tropfenweise mit einer Lösung von 293 mg (3.52 mmol, 2 Equivalent) Allylisocyanat in 2 ml Pyridin versetzt. Nach 6 Stunden werden weitere 2 Equivalente Allylisocyanat in 2 ml Pyridin zugegeben. Nach 30 Stunden werden nochmals 4 Equivalente Allylisocyanat zugegeben. Nach 5 Tagen wird die Reaktionslösung mit 50 ml Toluol verdünnt und dann vollständig eingeengt. Der Rückstand wird chromatographisch gereinigt (Kieselgel, Acetonitril / Wasser 1:1). Es verbleibt nach dem Entfernen des Lösungsmittels die im Titel genannte Verbindung.
R_{f} - Wert: 0.39 (CH₃CN / H₂O 7:3)
MS(FAB): 1240(M + Na)⁺, 1348(M + Na + Thioglycerin)⁺

### Beispiel 13

### 6-N-Allyl-amino-6-desoxy-β-cyclodextrin

Unter Argon werden 100 mg (78 µmol) 6-O-Monotoluolsulfonyl-β-cyclodextrin portionenweise unter Rühren zu 1.3 ml (17.5 mmol) Allylamin gegeben. Die resultierende Suspension wird für 4 Stunden auf 70 °C erwärmt. Das Reaktionsgemisch wird dann im Hochvakuum zur Trockene eingedampft. Der Rückstand wird in 2 ml Wasser gelöst und dann durch Zugabe von 10 ml Acetonitril ausgefällt. Es wird abgesaugt und mit wenig Acetonitril gewaschen. Nach dem Trocknen verbleiben 56 mg (61 %) farbloser Feststoff.
R_{f} - Wert: 0.10 (Ether/ NH₃[25% wässrig] / Isopropanol 5:6:6)
MS(FAB): 1174(M + H)⁺, 1281(M + Thioglycerin)⁺

### Beispiel 14

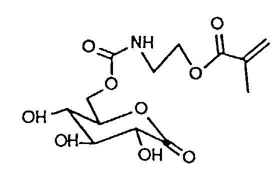

### 6-O-Carbamoyl-2-methylpropenoylethyl-gluconsäure-γ-lacton

Zu 5.0 g (28.1 mmol) D(+)-Gluconsäure-δ-lacton in 50 ml Pyridin werden langsam 4.4 g (28.1 mmol) IEM getropft. Es wird 2 Tage bei RT nachgerührt und dann wie in Beispiel 1 aufgearbeitet. Chromatographische Reinigung (Acetonitril / Wasser 9:1) ergibt 2.8 g (30 %) eines farblosen Pulvers.
MS(FAB): 334(M + H)⁺, 356(M + Na)⁺

### Beispiel 15

### 6-Allylamino-6-desoxy-methyl-β-D-glucopyranosid

Zu 100 mg 6-O-Tosyl-methyl-β-D-glucopyranosid wird 1 ml Allylamin gegeben und 12 Std. bei 40°C ausgerührt. Dann wird das Reaktionsgemisch im Hochvakuum eingeengt und anschliessend chromatographisch gereinigt (Acetonitril). Es verbleiben 30 mg (42 %) eines farblosen Oels.

### Beispiel 16

### 6-O-Carbamoyl-methacryloylethyl-α,β-maltose

In Analogie zu Beispiel 6 wurden 10 g (0.027 mol) Maltose in Pyridin mit 1 Equivalent IEM umgesetzt. Nach der Aufarbeitung und Reinigung verbleiben 2.9 g (22 %) als farbloses Pulver.
R_{f} - Wert: 0.32 (CH₃CN / H₂O 9:1)
MS(FAB): 496(M - H)⁻, 520(M + Na)⁺, 532(M + Cl)⁻

### Beispiel 17

### 6-O-Carbamoyl-methacryloylethyl-α,β-lactose

In Analogie zu Beispiel 16 wird die Titelverbindung in ähnlicher Ausbeute erhalten; ausgehend von Lactose und IEM in Pyridin.
MS(FAB): 498(M + H)⁺, 520(M + Na)⁺

### Beispiel 18

### 2-O-Carbamoyl-methacryloylethyl-1,6-anhydro-β-glucopyranose und Isomere

In Analogie zu Beispiel 8 werden 50 g (0.308 mol) 1,6-Anhydro-β-D-glucopyranosid mit 1 Equivalent IEM in Pyridin umgesetzt Nach dem Aufarbeiten und Reinigen verbleiben 44 g (45 %) eines Monoacrylatisomerengemisches.
MS(FAB): 318 (M + H)⁺

### Beispiel 19

### 6-O-Carbamoyl-methacryloylethyl-γ-cyclodextrin

In Analogie zu Beispiel 11 wird die Titelverbindung hergestellt aus 20 g (15.4 mmol) γ-CD und 7.2 g( 46.3 mmol) IEM in 250 ml Pyridin. Man erhält 4.47 g (20 %) eines farblosen Pulvers.
R_{f} - Wert: 0.36 (CH₃CN / H₂O 7:3)
MS(FAB): 1475 (M + Na)⁺

### Beispiel 20

### 6-O-Monoallylcarbamoyl-α-cyclodextrin

In Analogie zu Beispiel 12 werden 20 g α-Cyclodextrin mit 6.3 g (3.75 Equivalent) Allylisocyanat in Pyridin umgesetzt. Es verbleiben nach der Reinigung 4 g (19 %) eines farblosen Pulvers.
R_{f} - Wert: 0.15 (CH₃CN / H₂O 8:2)
MS(FAB): 1054(M - H)⁺

### Beispiel 21

### Synthese von Poly(6-O-Carbamoyl-methacryloylethyl-methyl-β-D-glucopyranosid) Homopolymer

Eine Lösung von 1.0 g 6-O-Carbamoyl-methacryloylethyl-methyl-β-D-glucopyranosid in 4 ml Wasser (entgast) wird unter Argon auf 0°C gekühlt. Dann wird 50 µl einer Ammoniumperoxodisulfatlösung (Konzentration dieser Persulfat-Lösung ist 10 mg/ml) und 50 µl einer Natriumdisulfitlösung (Konzentration dieser Disulfitlösung ist auch 10 mg/ml) zugegeben. Nach 2 Stunden wird die Reaktionslösung auf 400 ml Methanol gegossen, wobei das Polymer, 820 mg (82 %), als feiner weisser Feststoff ausfällt.

### Beispiel 22

### Synthese von Poly(6-O-Carbamoyl-methacryloylethyl-α,α-trehalose) Homopolymer

In einem Kolben wird unter Argon 1.0 g (2 mmol) 6-O-Carbamoyl-methacryloylethyl-α,α-trehalose in 4 ml entgastem Wasser (HPLC-Qualität) gelöst und auf 0°C gekühlt. Dazu wird je 100 µl einer Ammoniumperoxodisulfatlösung und 100 µl einer Natriumdisulfitlösung (Konzentration wie in Beispiel 21) gegeben. Die Polymerisation wird mit DC verfolgt (Acetonitril / Wasser 8:2). Nach 1 Stunde ist die Reaktion beendet. Die Reaktionslösung wird dann in 400 ml Methanol eingetropft, wobei ein weisser Feststoff ausfällt. Dieser wird abfiltriert, in Wasser aufgenommen und lyophilisiert. Das Homopolymer wird als farbloses amorphes Pulver, 900 mg (90 %), erhalten.

### Beispiel 23

### Synthese von Poly(6-O-Carbamoyl-methacryloylethyl-β,β-trehalose) Homopolymer

In Analogie zu Beispiel 22 wird 500 mg 6-O-Carbamoyl-methacryloylethyl-β,β-trehalose in 2 ml entgastem Wasser polymerisiert. Nach der Lyophilisation verbleiben 358 mg (72 %) weisser Feststoff.

### Beispiel 24

### Synthese von Poly(6-O-Carbamoyl-methacryloylethyl-α,β-maltotriose) Homopolymer

In Analogie zu Beispiel 22 wird 500 mg 6-O-Carbamoyl-methacryloylethyl-α,β-maltotriose in 2 ml entgastem Wasser und in Anwesenheit von 50 µl Ammoniumperoxodisulfatlösung und 50 µl einer Natriumdisulfitlösung polymerisiert. Nach der Lyophilisation verbleiben 371 mg weisser Feststoff.

### Beispiel 25

### Synthese von Poly(6-O-Carbamoyl-methacryloylethyl-α-cyclodextrin) Homopolymer

Zu einer Suspension von 100 mg (89 µmol) 6-O-Carbamoyl-methacryloylethyl-α-cyclodextrin in 0.2 ml Wasser (entgast) werden 20 µl Acetonitril gegeben. Unter Rühren wird in diese Lösung je 1 mg Ammoniumperoxodisulfat und 1 mg Natriumdisulfit eingetragen. Die Polymerisation wird mit DC verfolgt. Nach 20 Stunden wird die leicht trübe Reaktionslösung auf 40 ml Methanol gegossen. Der ausgefallene Feststoff wird abfiltriert, in Wasser aufgenommen und lyophilisiert. Es verbleiben 88 mg (88 %) Homopolymer.

### Beispiel 26

### Synthese von Poly(6-O-Carbamoyl-methacryloylethyl-β-cyclodextrin) Homopolymer

In einem Kolben werden 100 mg 6-O-Carbamoyl-methacryloylethyl-β-cyclodextrin in 0.6 ml trockenem Dimethylformamid (DMF) gelöst. Die Lösung wird dann klarfiltriert und mit 10 mg AIBN versetzt. Das Reaktionsgemisch wird entgast und dann unter Argon auf 80°C erwärmt. Man lässt über Nacht bei dieser Temperatur reagieren. Dann wird das Reaktionsgemisch in 100 ml Methanol eingerührt, wobei ein feiner weisser Feststoff ausfällt, der abfiltriert und getrocknet wird.

### Beispiel 27

### Synthese von Poly-Co(6-O-Carbamoyl-methacryloylethyl-α,α-trehalose Acrylamid) (Molverhältnis Trehalose / Acrylamid 3:1)

In einem Kolben werden 200 mg 6-O-Carbamoyl-methacryloylethyl-α,α-trehalose aus Beispiel 5 in entgastem Wasser unter Argon vorgelegt Hierzu wird 9.5 mg (0.134 mmol) Acrylamid gegeben. Dieses Gemisch wird mit Argon gespült, auf 0°C abgekühlt und dann mit je 10 µl Ammoniumperoxodisulfatlösung und 10 µl Natriumdisulfitlösung (Konzentration wie in Beispiel 21) versetzt. Das Reaktionsgemisch wird 3 Tage gerührt. Dann wird mit 100 ml Methanol versetzt, wobei ein weisser Feststoff ausfällt, der abfiltriert und getrocknet wird. Es verbleiben 50 mg Copolymer.

### Beispiel 28

### Synthese von Poly-Co(6-O-Carbamoyl-methacryloylethyl-α,α-trehalose Acrylamid) (Molverhältnis Trehalose / Acrylamid 1:3)

In Analogie zu Beispiel 27 werden 200 mg Trehalosederivat aus Beispiel 5 mit 85.7 mg (1.2 mmol) Acrylamid copolymerisiert. Nach der Aufarbeitung wie in Beispiel 27 verbleiben 238 mg (83 %) Polymer.

### Beispiel 29

### Synthese von Poly-Co(6-O-Carbamoyl-methacryloylethyl-α,α-trehalose Hydroxyethylmethacrylat(HEMA)) (Molverhältnis Trehalose / HEMA 1:3)

Eine Lösung von 200 mg 6-O-Carbamoyl-methacryloylethyl-α,α-trehalose aus Beispiel 5 in 0.77 ml Wasser wird mit 137 mg (1.2 mmol) HEMA (technische Qualität) versetzt und analog zu Beispiel 27 polymerisiert. Nach 3 Stunden entsteht ein wasserunlösliches Gel.

### Beispiel 30

### Synthese von Poly-Co(6-O-Carbamoyl-methacryloylethyl-α,α-trehalose Hydroxyethylmethacrylat (HEMA)) (Molverhältnis Trehalose / HEMA 3:1)

In Analogie zu Beispiel 29 werden 200 mg 6-O-Carbamoyl-methacryloylethyl-α,α-trehalose und 15.3 mg (0.134 mmol) HEMA polymerisiert. Es entsteht kein Gel, das Reaktionsgemisch bleibt eine klare Lösung. Diese wird mit 100 ml Methanol verdünnt, wobei ein Feststoff ausfällt. Dieser wird abfiltriert. Es verbleiben 160 mg (73 %).

### Beispiel 31

### Synthese von Poly-Co(6-N-Allyl-amino-6-desoxy-β-cyclodextrin Acrylamid) (Molverhältnis β-CD / Acrylamid 1:1)

100 mg (0.085 mmol) 6-N-Allyl-amino-6-desoxy-β-cyclodextrin aus Beispiel 13 werden in 2 ml entgastem Wasser und unter Argon vorgelegt. Hierzu wird 18 mg (0.26 mmol) Acrylamid und 26 µl Tetramethylethylendiamin gegeben. Bei 0°C wird dann portionenweise 26 mg Ammoniumperoxodisulfat zugegeben. Nach 24 Stunden wird die Reaktionslösung in 40 ml Methanol eingerührt. Dabei fällt ein weisser Feststoff aus, der abfiltriert, in Wasser aufgenommen und anschliessend lyophilisiert wird. Es verbleiben 33 mg.

### Beispiel 32

Synthese von Poly-Co(6-N-Allyl-amino-6-desoxy-β-cyclodextrin Acrylamid) (Molverhältnis β-CD / Acrylamid 1:3)

In Analogie zu Beispiel 31 wird im Verhältnis 1:3 copolymerisiert. Es verbleiben nach dem Lyophilisieren 27 mg.

### Beispiel 33

### Synthese von Poly-Co(6-O-Carbamoyl-methacryloylethyl-α,α-trehalose N-Vinyl-2-pyrrolidon (NVP)) (Molverhältnis 1:1)

In Analogie zu Beispiel 30 werden Trehalose-monoacrylat aus Beispiel 5 und NVP in Wasser copolymerisiert. Man erhält ein Gel.

### Beispiel 34

Synthese weiterer Copolymere in Analogie zu Beispiel 27 in tabellarischer Uebersicht (Ausbeute jeweils 75 %):

| Kohlenhydratderivat | Comonomer | Verhältnis |
|---|---|---|
| Trehalosederivat aus Beisp. 5 | N,N-Dimethylacrylamid | 3 : 1 |
| Trehalosederivat aus Beisp. 5 | Nippon Blemer® GLM | 3 : 1 |

### Beispiel 35

### Photopolymerisation

In einem braunen Rundkolben werden 1 g eines Kohlenhydratmonomers in 4 ml entgastem Wasser gelöst. Dazu gibt man eine entsprechende Menge eines verwendeten Comonomers und eine entsprechende Menge Photoinitiator. Restluft wird durch wiederholtes Evakuieren und Belüften mit Argon entfernt. Dann gibt man diese Lösung in Förmchen (z.B. mit Kontaktlinsengeometrie) und bestrahlt unter Argon mit UV-Licht geeigneter Wellenlänge. Die so erhaltenen Rohlinge werden durch Extraktion mit Wasser von den Restmonomeren befreit und anschliessend auf ihre Eigenschaften überprüft.

### Beispiel 36

Gewaschene und getrocknete STD™ Kontaktlinsen (CIBA Vision, Atlanta, Tefilcon) auf der Basis von quervernetztem Poly-HEMA, werden in einer Lösung von 5 ml THF, 5 ml Diethylether, 0.2 g Diisophorondiisocyanat (IPDI) und 10 mg Dibutylzinndilaurat (DBTDL) durchtränkt. Man belässt die Linsen dort bei RT unter Stickstoff für 12 Stunden. Danach werden die Linsen mit Aceton gewaschen, getrocknet und dann in einer 0.5 % Lösung der Polymere aus den Beispielen 21, 22 und 25 in DMSO, zusätzlich enthaltend 5 % LiCl und 0.1 % DBTDL als Katalysator, getränkt. Man belässt dort die Linsen für 12 Stunden bei 25 - 40°C, wäscht dann gut mit Wasser und trocknet die Linsen im Anschluss. Dann werden die statischen Kontaktwinkel (CA) der unbehandelten und der behandelten Kontaktlinsen mit einem G 40 System gemessen (Krüss GmbH, Hamburg Germany).

| behandelt mit Polymer | statische Kontaktwinkel (CA) |
|---|---|
| aus Beispiel 21 | 58° |
| aus Beispiel 22 | 41° |
| aus Beispiel 25 | 32° |
| | |
| unbehandelte Linse | 78° |

### Beispiel 37

### Herstellung einer Plasma-modifizierten Polymeroberfläche

Ein Silikonfilm, der hergestellt wurde durch UV-Härtung von Silicon PS 2067 (Hüls America Inc., Bristol, USA), wird in einen RF-GDP (radio frequency glow discharge plasma) Reaktor gegeben. Der Reaktor wird auf 0.1 mbar evakuiert. Dann wird der Silikonfilm einem Sauerstoffplasma bei 40 Watt Leistung und einem Sauerstoffgasfluss von 10 cm³/ min (STP) für 30 Sekunden ausgesetzt. Danach wird der Reaktor belüftet.

### Beispiel 38

Der im Plasma behandelte Film aus Beispiel 37 wird in einem Exsikkator über 5 ml Toluol-2,4-diisocyanat plaziert, dann wird auf 0.008 mbar evakuiert und auf 50 °C erwärmt. Der Film wird für 2.5 Stunden im Exsikkator belassen (Derivatisierung mit Toluol-2,4-diisocyanat), dann wird auf RT abgekühlt. Der Film wird entnommen und mit Aceton gewaschen. Danach wird der so vorbehandelte Film für 8 Stunden in eine DMSO-Lösung getaucht, enthaltend das Trehalosepolymerisat aus Beispiel 22 und 5 % LiCl. Der so modifizierte Film wird dann mit Wasser gewaschen, getrocknet und analysiert.

### Beispiele 39 - 41

Beispiel 39: Ein Polybutadien-Film wird gemäss Beispiel 37 mit einem Sauerstoffplasma behandelt. Die anschliessende Exposition in Toluol-2,4-diisocyanat gemäss Beispiel 38 beträgt 2.5 Stunden. Die Behandlung mit dem Trehalosepolymerisat beträgt 8 Stunden.

Beispiel 40: Ein Poly-HEMA-Film hergestellt aus einer Lösung enthaltend HEMA (92 %), Ethylenglykoldimethacrylat (5 %) und einen Photoinitiator Irgacur 184 (3 %), durch Giessen auf eine Folanorm Folie und UV-Bestrahlung. Die Exposition in Toluol-2,4-diisocyanat gemäss Beispiel 38 beträgt 6 Stunden. Die Behandlung mit dem Trehalosepolymerisat beträgt ebenfalls 8 Stunden.

Beispiel 41: Ein Polyvinylalkohol (PVA)-Film wird hergestellt aus einer DMSO-Lösung von PVA 72 000 (Fluka) und IPDI (Aldrich) durch Giessen auf eine Folanorm Folie und erhitzen auf 70°C für 2 Stunden. Weiterbehandlung des Films gemäss Beispiel 40.

Die Bestimmung der Kontaktwinkel (CA) erfolgt wie in Beispiel 36 beschrieben.

| Beispiel | Polymerfilm | CA vor der Behandlung (°) | CA nach der Behandlung (°) |
|---|---|---|---|
| 38 | Silikon | 100.4 | 51.6 |
| 39 | Polybutadien | 79.5 | 49.3 |
| 40 | Poly-HEMA | 78.4 | 44.6 |
| 41 | PVA | 47.1 | 23.8 |

### Beispiel 42 Herstellung von

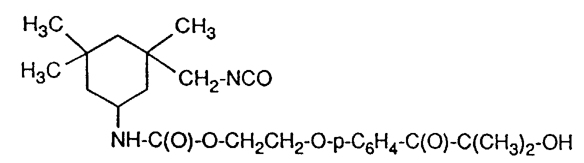

In einem 500 ml Kolben mit Rückflusskühler, Thermometer, Rührer und Stickstoffeinleitungsrohr wird unter Stickstoff eine Lösung von 11,125 g (0,05 Mol) frisch destilliertem Isophorondiisocyanat (IPDI) in 50 ml trockenem Methylenchlorid mit einer Lösung von 11,2 g (0,05 Mol) 4'-(β-Hydroxyethoxy)-2-hydroxyprop-2-yl-phenon (Darocure 2959®) in 300 ml trockenem Methylenchlorid gemischt und nach Zugabe von 20 mg Dibutyl-zinn-dilaurat als Katalysator 48 Stunden bei Raumtemperatur gerührt. Der Verlauf der Umsetzung wird mittels Dünnschichtchromatographie auf Kieselgelplatten (60 F₂₅₄, Art. 5719 Merck) verfolgt (Laufmittel: Toluol/Acetonitril 7:3). Das entstandene Produkt wird durch Säulenchromatographie an Kieselgel 60 (Eluent Toluol/Acetonitril 7:3) von geringen Mengen an nicht umgesetztem Darocure 2959 und Bisaddukt von IPDI befreit. Nach Eindampfen der reinen Fraktionen am Rotationsverdampfer wird ein farbloses Oel erhalten, das beim Abkühlen auf -16°C langsam kristallisiert und anschliessend aus trockenem Diethylether umkristallisiert wird. Es werden 15,6 g eines weissen kristallinen Produktes erhalten (70 % d. Th.), das einen Schmelzpunkt von 76°C aufweist.

Der Isocyanatgehalt des Produktes wird durch Titration mit Dibutylamin in Toluol ermittelt: berechnet 2,242 mVal/g, gefunden 2,25 mVal/g.

Die Methode ist in "Analytical Chemistry of Polyurethanes" (High Polymer Series XVI/Part III, D.S. David + H.B. Staley editors, Interscience Publishers, New York 1969 p. 86) beschrieben.

### Beispiel 43 Oberflächenreaktion einer Kontaktlinse

Kontaktlinsen aus vernetzten Polyhydroxyethylmethacrylaten (Poly-HEMA) werden auf der Oberfläche mit einer Lösung der Verbindung aus Beispiel 42 in Tetrahydrofuran (Konzentration 5 %) beziehungsweise Diethylether benetzt. Die behandelten Kontaktlinsen werden unter trockenem Stickstoff 16 Stunden bei Raumtemperatur gelagert. Danach werden die Kontaktlinsen 8 Stunden mit Aceton gewaschen und dann im Hochvakuum getrocknet.

### Beispiel 44 Modifikation der Oberfläche einer Kontaktlinse.

Gemäss Beispiel 43 behandelte Kontaktlinsen werden in eine wässrige Lösung des Monomeren aus Beispiel 5 getaucht und dann durch mehrfaches Evakuieren und Entlasten mit Stickstoff von Sauerstoff befreit. Dann wird mit einer Hg-Hochdrucklampe (Photoresistbelichter 82420, Oniel, 2000 W) unter Stickstoff 2 mal 2 Minuten bestrahlt. Die Kontaktlinsen werden darauf mit destilliertem Wasser gewaschen und im Hochvakuum getrocknet. Die Kontaktlinsen zeigen vor und nach der Behandlung die folgenden Werte für die Kontaktwinkel (advancing und receding angle) und die Kontaktwinkel-Hysterese. Die Zahlenwerte zeigen die verbesserte Hydrophilie, das gute Wasserrückhaltevermögen und die vollständige Beschichtung der so erzeugten Oberfläche.

| Kontaktlinse | Advancing angle | Receding angle | Hysterese (°) | Retention Wasserfilm |
|---|---|---|---|---|
| Poly-HEMA unbehandlet | 78.4° | 33.3° | 45.1° | ca. 10 sec |
| | | | | |
| Poly-HEMA behandelt | 41.2° | 29.9° | 11.3° | > 2 min |

### Beispiel 45

### Beschichtung eines Polymersubstrates

Eine Folie (2 cm · 2 cm) aus Poly-Hydroxyethylmathacrylat (Poly-HEMA) und 3% Diethylenglykoldiacrylat (DEGDA) als Quervernetzer wird in einem Plasmareaktor vorgelegt. Die Reaktorkammer wird dann unter Glimmentladungsbedingungen mit 1,2-Diaminocyclohexan als Plasmagas unter den nachfolgenden Bedingungen beschickt:
Radiofrequenz von 27.12 MHz, 3 Watt Leistung, 0.5 mbar (50 Pa) Druck, Arbeitsgasflussrate 3.65 cm³/ min (STP), Verweilzeit der Folie im Reaktor beträgt 5 Minuten. Die so behandelte Folie wird dann für 8 Std. bei Raumtemperatur (RT) und unter Stickstoff in eine THF/ Diethylether-Lösung (1:2) enthaltend 1 Gew.% der Verbindung aus Beispiel 42 und eine katalytische Menge Dibutylzinndilaurat (DBTDL) getaucht. Dabei wird der reaktive Photoinitiator an die durch die Plasmabehandlung erzeugten Aminogruppen auf der Folienoberfläche gebunden. Die Folie wird anschliessend 3 Stunden in THF gewaschen und dann für 3 Std. im Vakuum getrocknet. Die getrocknete Folie wird dann in eine gerührte 15 %-ige wässrige Lösung des Trehalosemonomeren aus Beispiel 5 gegeben und anschliessend für 4 Minuten mit einer Hg-Hochdrucklampe (2000 Watt) beidseitig bestrahlt. Man wäscht danach die beschichtete Folie mehrmals in destilliertem Wasser und misst dann die Kontaktwinkel und die Wasserretentionszeiten.

| Poly-HEMA | Advancing angle | Receding angle | Wasserretention Zeit |
|---|---|---|---|
| unbehandelt | 82.8° | 47.4° | ca. 10 sec |
| behandelt | 41.2° | 29.9° | > 2 min |

### Beispiel 46

Eine Folie (2 cm · 2 cm) aus Silikongummi, hergestellt durch Vernetzen von 2 mol Vinylpolysiloxan (Silopren U Additiv V 200, Bayer) mit 4 mol H-Siloxan (K-3272, Goldschmidt), wird in einem Plasmareaktor (gemäss Beispiel 45) unter Glimmentladungsbedingungen mit 1,2-Diaminocyclohexan als Plasmagas behandelt. Der so vorbehandelte Silikonfilm wird dann für 3 Stunden bei RT unter Stickstoff in eine Acetonitril-Lösung getaucht, enthaltend 1 % des koreaktiven Photoinitiators aus Beispiel 42 und 10 mg DBTDL als Katalysator. Der so behandelte Film wird dann mit Acetonitril gewaschen und im Vakuum getrocknet. Danach taucht man diesen Film in eine wässrige Lösung, enthaltend 1.5 g des Monomeren aus Beispiel 5 in 10 ml Wasser. Im Anschluss daran wird der so beschichtete Film für 4 Minuten mit einer Hg-Hochdrucklampe (2000 Watt) beidseitig bestrahlt. Man wäscht den beschichteten Film mehrmals in destilliertem Wasser und misst dann die Kontaktwinkel und die Wasserretentionszeiten.

| Polymer | Advancing angle | Receding angle | Wasserretention Zeit |
|---|---|---|---|
| Silikon unbehandelt | 122.4° | 101.8° | < 3 sec |
| Silikon behandelt | 62.7° | 37.6° | > 20 sec |

### Beispiel 47

In Analogie zu Beispiel 45 wird eine gewaschene und getrocknete Weicon Kontaklinse (CIBA-Vision Atlanta) ebenfalls mit dem Photoinitiator aus Beispiel 42 und dem Monomer aus Beispiel 5 beschichtet. Man wäscht die behandelte Linse mehrmals in destilliertem Wasser und misst dann die Kontaktwinkel und die Wasserretentionszeiten.

| Weicon Kontaktlinse | Advancing angle | Receding angle | Wasserretention Zeit |
|---|---|---|---|
| unbehandelt | 78.4° | 44.3° | ca. 12 sec |
| behandelt | 59.2° | 34.6° | 1.8 min |

### Beispiel 48

Eine gemäss Beispiel 46 hergestellte Silikonkontaktlinse wird gemäss Beispiel 46 beschichtet. Man wäscht dann die behandelte Linse mehrmals in destilliertem Wasser und misst dann die Kontaktwinkel und die Wasserretentionszeiten.

| Silikonkontaktlinse | Advancing angle | Receding angle | Wasserretention Zeit |
|---|---|---|---|
| unbehandelt | 120.3° | 100.8° | < 3 sec |
| behandelt | 59.9° | 39.4° | > 20 sec |

## Patentansprüche

1. Eine Verbindung der Formel I,
R¹-(COO-Alk)ₘ-(OCONH-R)ₙ-(NHCO)ₚ-Y-Z (I)
worin R¹ eine radikalisch polymerisierbare Kohlenwasserstoffgruppe bedeutet; m, n und p die Werte 0 oder 1 haben; Alk Alkylen mit bis zu 10 C-Atomen bedeutet; R ein Diradikal mit bis zu 20 C-Atomen eines organischen Diisocyanates darstellt; Z für einen um eine einzelne primäre Hydroxylgruppe verminderten einwertigen Rest eines Mono-, Di- oder Trisaccharides, eines Oligosaccharides oder eines Cyclodextrins (CD) steht; und Y -O- oder -NH- darstellt; unter der Bedingung, dass wenn p null ist, m und n ebenfalls null sind und Y -NH- bedeutet; ferner unter der Bedingung, dass m, n und p null sind, wenn Y -NH- bedeutet.

2. Eine Verbindung gemäss Anspruch 1, dadurch gekennzeichnet, dass Y -O- bedeutet.

3. Eine Verbindung gemäss einem der Ansprüche 1 oder 2, dadurch gekennzeichnet, dass m=1, p=1 und n=0 ist.

4. Eine Verbindung gemäss Anspruch 1, dadurch gekennzeichnet, dass m und n null sind.

5. Eine Verbindung gemäss Anspruch 1, dadurch gekennzeichnet, dass R' für Alkenyl mit 2 bis 12 C-Atomen steht.

6. Eine Verbindung gemäss Anspruch 1, dadurch gekennzeichnet, dass Alk ein Niederalkylen mit bis zu 7 C-Atomen ist.

7. Eine Verbindung gemäss Anspruch 1, dadurch gekennzeichnet, dass das Diradikal R Niederalkylen, Arylen, eine gesättigte bivalente cycloaliphatische Gruppe mit 6 bis 10 Kohlenstoffatomen, Alkylenarylen, Arylenalkylen oder Arylenalkylenarylen bedeutet.

8. Eine Verbindung gemäss Anspruch 1, dadurch gekennzeichnet, dass der Rest Z sich von einem Monosaccharid ableitet, das ausgewählt ist unter einer Aldopentose, Aldohexose, Ketopentose und Ketohexose.

9. Eine Verbindung gemäss Anspruch 1, dadurch gekennzeichnet, dass der Rest Z sich von einem Disaccharid ableitet, das ausgewählt ist unter einer Trehalose, Maltose, Isomaltose, Cellobiose, Gentiobiose, Saccharose und Lactose.

10. Eine Verbindung gemäss Anspruch 1, dadurch gekennzeichnet, dass der Rest Z sich von einem Trisaccharid ableitet, das ausgewählt ist unter einer Raffinose, Panose und Maltotriose.

11. Eine Verbindung gemäss Anspruch 1, dadurch gekennzeichnet, dass der Rest Z sich von einem Oligosaccharid ableitet, das ausgewählt ist unter einer Maltotetraose, Maltohexaose und Chitoheptaose.

12. Eine Verbindung gemäss Anspruch 1, dadurch gekennzeichnet, dass der Rest Z sich von einem Cyclodextrin ableitet, das ausgewählt ist unter einem α-, β- und γ- Cyclodextrin.

13. Eine Verbindung gemäss Anspruch 1, dadurch gekennzeichnet, dass der Rest Z sich von einem Monosaccharid ableitet, das ausgewählt ist unter einer Aldohexose und Ketohexose.

14. Eine Verbindung gemäss Anspruch 1, dadurch gekennzeichnet, dass der Rest Z sich von einem Disaccharid ableitet, das ausgewählt ist unter einer α,α-, α,β- und β,β-Trehalose.

15. Eine Verbindung gemäss Anspruch 1, dadurch gekennzeichnet, dass der Rest Z sich von einem Cyclodextrin ableitet, das ausgewählt ist unter einem α- und β- Cyclodextrin.

16. Eine Verbindung gemäss Anspruch 1, dadurch gekennzeichnet, dass der Rest Z sich von einem Monosaccharid ableitet, das für ein 1-Alkylglucosid steht.

17. Eine Verbindung gemäss Anspruch 1, dadurch gekennzeichnet, dass der Rest Z sich von einem Disaccharid ableitet, das für eine α,α-Trehalose steht.

18. Eine Verbindung gemäss Anspruch 1, dadurch gekennzeichnet, dass der Rest Z sich von einem Cyclodextrin ableitet, das für ein α- Cyclodextrin steht.

19. Eine Verbindung gemäss Anspruch 1, dadurch gekennzeichnet, dass R¹ Alkenyl mit 2 bis 8 C-Atomen bedeutet; m und p den Wert 1 haben und n null ist; Alk Niederalkylen mit bis zu 4 C-Atomen bedeutet; Y -O- darstellt; und der Rest Z sich von einem Saccharid ableitet, das für ein 1-Alkylglucosid, eine a,a-Trehalose oder ein α- Cyclodextrin steht.

20. Eine Verbindung gemäss Anspruch 1, dadurch gekennzeichnet, dass R¹ Alkenyl mit 2 bis 8 C-Atomen bedeutet; m, n und p null sind; Y -NH- darstellt; und der Rest Z sich von einem Saccharid ableitet, das für ein 1-Alkylglucosid, eine α,α-Trehalose oder ein α- Cyclodextrin steht.

21. Eine Verbindung gemäss Anspruch 1, dadurch gekennzeichnet, dass R¹ Alkenyl mit 2 bis 8 C-Atomen bedeutet; m und n den Wert null haben und p 1 ist; und der Rest Z sich von einem Saccharid ableitet, das für ein 1-Alkylglucosid, eine α,α-Trehalose oder ein α- Cyclodextrin steht.

22. Eine Verbindung gemäss Anspruch 2, dadurch gekennzeichnet, dass R¹ Alkenyl mit 2 bis 4 C-Atomen bedeutet; und Alk ein Alkylen mit bis zu 4 C-Atomen bedeutet.

23. Verfahren zur Herstellung einer Verbindung der Formel (I) gemäss der Definition in Anspruch 1, dadurch gekennzeichnet, dass ein Saccharid der Formel (II)
Z-X (II)
worin Z für einen um eine einzelne primäre Hydroxylgruppe verminderten einwertigen Rest eines Mono-, Di- oder Trisaccharides, eines Oligosaccharides oder eines Cyclodextrins (CD) steht; und X für eine reaktionsfähige Gruppe steht, die an den um die primäre Hydroxylgruppe verminderten einwertigen Rest eines Saccharides Z geknüpft ist; mit einem Derivat der Formel (III)
R¹-(COO-Alk)ₘ-(OCONH-R)ₙ-NCO (III)
oder mit einem Derivat der Formel (IV)
R¹-NH₂ (IV)
umgesetzt wird, wobei R¹ eine radikalisch polymerisierbare Kohlenwasserstoffgruppe bedeutet; m, n und p die Werte 0 oder 1 haben; Alk Alkylen mit bis zu 10 C-Atomen bedeutet; und R ein Diradikal mit bis zu 20 C-Atomen eines organischen Diisocyanates darstellt.

24. Ein Polymer enthaltend ein Polymerisationsprodukt von mindestens einer Verbindung der Formel (I) gemäss der Definition in Anspruch 1 und gegebenenfalls von mindestens einem weiteren davon verschiedenartigen vinylischen Comonomer (a), wobei ein Polymer ausgenommen ist, das durch Umsetzung von 6-Iodo-6-desoxy-β-cyclodextrin mit Polyallylamin erhältlich ist.

25. Ein Polymer gemäss Anspruch 24, dadurch gekennzeichnet, dass Comonomer (a) abwesend ist und es sich um ein Homopolymer handelt.

26. Ein Polymer gemäss Anspruch 24, dadurch gekennzeichnet, dass der Gewichtsanteil bezüglich Gesamtpolymer einer Verbindung der Formel (I) im Bereich von 100 - 0.5 % liegt.

27. Ein Polymer gemäss Anspruch 24, dadurch gekennzeichnet, dass das Comonomer (a) hydrophob ist, und ausgewählt ist unter C₁-C₁₈-Alkyl- und C₃-C₁₈-Cycloalkylacrylaten und -methacrylaten, C₃-C₁₈-Alkylacrylamiden und -methacrylamiden, Acrylnitril, Methacrylnitril, Vinyl-C₁-C₁₈-alkanoaten, C₂-C₁₈-Alkenen, C₂-C₁₈-Haloalkenen, Styrol, Niederalkylstyrolen, Niederalkylvinylethern, C₂-C₁₀-Perfluoralkyl-acrylaten und -methacrylaten oder entsprechend partiell fluorierten Acrylaten und Methacrylaten, C₃-C₁₂-Perfluoralkyl-ethyl-thiocarbonylaminoethyl-acrylaten und -methacrylaten, Acryloxy und Methacryloxy-alkylsiloxanen, N-Vinylcarbazol und C₁-C₁₂-Alkylestern der Maleinsäure, Fumarsäure, Itaconsäure und Mesaconsäure.

28. Ein Polymer gemäss Anspruch 24, dadurch gekennzeichnet, dass das Comonomer (a) hydrophil ist, und ausgewählt ist unter durch Hydroxy substituierten Niederalkylacrylaten und -methacrylaten, Acrylamid, Methacrylamid, Niederalkylacrylamiden und -methacrylamiden, ethoxylierten Acrylaten und Methacrylaten, durch Hydroxy substituierten Niederalkylacrylamiden und Methacrylamiden, durch Hydroxy substituierten Niederalkylvinylethern, Natriumvinylsulfonat, Natriumstyrolsulfonat, 2-Acrylamido-2-methylpropansulfonsäure, N-Vinylpyrrol, N-Vinyl-2-pyrrolidon, 2- und 4-Vinylpyridin, vinylisch ungesättigten Carbonsäuren mit insgesamt 3 bis 5 Kohlenstoffatomen, Aminoniederalkyl- (wobei der Begriff "Amino" auch quaternäres Ammonium umfasst), Mononiederalkylamino-niederalkyl- und Diniederalkylamino-niederalkylacrylaten und -methacrylaten und Allylalkohol.

29. Ein polymeres Netzwerk, bestehend im wesentlichen aus einem Polymer gemäss Anspruch 24 in vernetzter Form.

30. Ein polymeres Netzwerk, bestehend im wesentlichen aus einem Polymer gemäss Anspruch 25 in vernetzter Form.

31. Verfahren zur Herstellung eines polymeren Netzwerkes gemäss einem der Ansprüche 29 oder 30, dadurch gekennzeichnet, dass man ein Polymer gemäss einem der Ansprüche 24 oder 25 vernetzt.

32. Formkörper bestehend im wesentlichen aus einem Polymer gemäss Anspruch 24.

33. Formkörper bestehend im wesentlichen aus einem polymeren Netzwerk gemäss einem der Ansprüche 29 oder 30.

34. Formkörper gemäss einem der Ansprüche 32 oder 33, dadurch gekennzeichnet, dass es sich um eine Kontaktlinse handelt.

35. Biomedizinischer Artikel, bestehend im wesentlichen aus einem Polymer gemäss einem der Ansprüche 24 oder 25.

36. Biomedizinischer Artikel, bestehend im wesentlichen aus einem polymeren Netzwerk gemäss einem der Ansprüche 29 oder 30.

37. Kontaktlinse, bestehend im wesentlichen aus einem Polymer gemäss einem der Ansprüche 24 oder 25.

38. Kontaktlinse, bestehend im wesentlichen aus einem polymeren Netzwerk gemäss einem der Ansprüche 29 oder 30.

39. Verwendung einer Verbindung der Formel (I) gemäss Anspruch 1 zur Beschichtung einer Körperoberfläche.

40. Verwendung eines Polymers gemäss einem der Ansprüche 24 oder 25 zur Beschichtung einer Körperoberfläche.

41. Verwendung gemäss Anspruch 39 oder 40, dadurch gekennzeichnet, dass die Körperoberfläche eine Polymersubstratoberfläche ist.

42. Verwendung gemäss Anspruch 39 oder 40, dadurch gekennzeichnet, dass die Körperoberfläche eine Kontaktlinsenoberfläche ist.

43. Verwendung eines Polymers gemäss einem der Ansprüche 24 oder 25 als pharmazeutisches Wirkstoff-Abgabesystem.

44. Verwendung eines polymeren Netzwerkes gemäss einem der Ansprüche 29 oder 30 als pharmazeutisches Wirkstoff-Abgabesystem.

## Claims

1. A compound of formula I
R¹-(COO-Alk)ₘ-(OCONH-R)ₙ-(NHCO)ₚ-Y-Z (I)
wherein
R¹ is a radically polymerisable hydrocarbon group;
m, n and p are 0 or 1;
Alk is alkylene having up to 10 carbon atoms;
R is a diradical, having up to 20 carbon atoms, of an organic diisocyanate;
Z is a monovalent radical, minus a single primary hydroxy group, of a mono-, di- or tri-saccharide, of an oligosaccharide or of a cyclodextrin (CD); and
Y is -O- or -NH-;
with the proviso that when p is zero, m and n are also zero and Y is -NH-; and with the proviso that m, n and p are zero when Y is -NH-.

2. A compound according to claim 1, wherein Y is -O-.

3. A compound according to either claim 1 or claim 2, wherein m is 1, p is 1 and n is 0.

4. A compound according to claim 1, wherein m and n are zero.

5. A compound according to claim 1, wherein R¹ is alkenyl having from 2 to 12 carbon atoms.

6. A compound according to claim 1, wherein Alk is lower alkylene having up to 7 carbon atoms.

7. A compound according to claim 1, wherein the diradical R is lower alkylene, arylene, a saturated divalent cycloaliphatic group having from 6 to 10 carbon atoms, alkylenearylene, arylenealkylene or arylenealkylenearylene.

8. A compound according to claim 1, wherein the radical Z is derived from a monosaccharide selected from an aldopentose, aldohexose, ketopentose and ketohexose.

9. A compound according to claim 1, wherein the radical Z is derived from a disaccharide selected from a trehalose, maltose, isomaltose, cellobiose, gentiobiose, saccharose and lactose.

10. A compound according to claim 1, wherein the radical Z is derived from a trisaccharide selected from a raffinose, panose and maltotriose.

11. A compound according to claim 1, wherein the radical Z is derived from an oligosaccharide selected from a maltotetraose, maltohexaose and chitoheptaose.

12. A compound according to claim 1, wherein the radical Z is derived from a cyclodextrin selected from an α-, β- and γ-cyclodextrin.

13. A compound according to claim 1, wherein the radical Z is derived from a monosaccharide selected from an aldohexose and ketohexose.

14. A compound according to claim 1, wherein the radical Z is derived from a disaccharide selected from an α,α-, α,β- and β,β-trehalose.

15. A compound according to claim 1, wherein the radical Z is derived from a cyclodextrin selected from an α- and β-cyclodextrin.

16. A compound according to claim 1, wherein the radical Z is derived from a monosaccharide that is a 1-alkyl glucoside.

17. A compound according to claim 1, wherein the radical Z is derived from a disaccharide that is an α,α-trehalose.

18. A compound according to claim 1, wherein the radical Z is derived from a cyclodextrin that is an α-cyclodextrin.

19. A compound according to claim 1, wherein R¹ is alkenyl having from 2 to 8 carbon atoms; m and p are 1 and n is zero; Alk is lower alkylene having up to 4 carbon atoms; Y is -O-; and the radical Z is derived from a saccharide that is a 1-alkyl glucoside, an α,α-trehalose or an α-cyclodextrin.

20. A compound according to claim 1, wherein R¹ is alkenyl having from 2 to 8 carbon atoms; m, n and p are zero; Y is -NH-; and the radical Z is derived from a saccharide that is a 1-alkyl glucoside, an α,α-trehalose or an α-cyclodextrin.

21. A compound according to claim 1, wherein R¹ is alkenyl having from 2 to 8 carbon atoms; m and n are zero and p is 1; and the radical Z is derived from a saccharide that is a 1-alkyl glucoside, an α,α-trehalose or an α-cyclodextrin.

22. A compound according to claim 2, wherein R¹ is alkenyl having from 2 to 4 carbon atoms and Alk is alkylene having up to 4 carbon atoms.

23. A process for the preparation of a compound of formula (I) as defined in claim 1, which process comprises reacting a saccharide of formula (II)
Z-X (II),
wherein Z is a monovalent radical, minus a single primary hydroxy group, of a mono-, di- or tri-saccharide, of an oligosaccharide or of a cyclodextrin (CD), and X is a reactive group linked to the monovalent radical, minus the primary hydroxy group, of a saccharide Z, with a derivative of formula (III)
R¹-(COO-Alk)ₘ-(OCONH-R)ₙ-NCO (III)
or with a derivative of formula (IV)
R¹-NH₂ (IV),
wherein R¹ is a radically polymerisable hydrocarbon group; m, n and p are 0 or 1; Alk is alkylene having up to 10 carbon atoms; and R is a diradical, having up to 20 carbon atoms, of an organic diisocyanate.

24. A polymer comprising a polymerisation product of at least one compound of formula (I) as defined in claim 1 and optionally of at least one other vinylic comonomer (a) that is different therefrom, with the exception of a polymer that is obtainable by reaction of 6-iodo-6-deoxy-β-cyclodextrin with polyallylamine.

25. A polymer according to claim 24, wherein comonomer (a) is absent and the polymer is a homopolymer.

26. A polymer according to claim 24, wherein the proportion by weight, based on the total polymer, of a compound of formula (I) is in the range of from 100 to 0.5 %.

27. A polymer according to claim 24, wherein the comonomer (a) is hydrophobic and is selected from C₁-C₁₈alkyl and C₃-C₁₈cycloalkyl acrylates and methacrylates, C₃-C₁₈alkylacrylamides and -methacrylamides, acrylonitrile, methacrylonitrile, vinyl-C₁-C₁₈alkanoates, C₂-C₁₈alkenes, C₂-C₁₈haloalkenes, styrene, lower alkylstyrenes, lower alkyl vinyl ethers, C₂-C₁₀perfluoroalkyl acrylates and methacrylates or correspondingly partially fluorinated acrylates and methacrylates, C₃-C₁₂perfluoroalkyl-ethyl-thiocarbonylaminoethyl acrylates and methacrylates, acryloxy- and methacryloxyalkylsiloxanes, N-vinylcarbazole, and C₁-C₁₂alkyl esters of maleic acid, fumaric acid, itaconic acid and mesaconic acid.

28. A polymer according to claim 24, wherein the comonomer (a) is hydrophilic and is selected from hydroxy-substituted lower alkyl acrylates and methacrylates, acrylamide, methacrylamide, lower alkyl-acrylamides and -methacrylamides, ethoxylated acrylates and methacrylates, hydroxy-substituted lower alkyl-acrylamides and -methacrylamides, hydroxy-substituted lower alkyl vinyl ethers, sodium vinylsulfonate, sodium styrenesulfonate, 2-acrylamido-2-methylpropanesulfonic acid, N-vinylpyrrole, N-vinyl-2-pyrrolidone, 2- and 4-vinylpyridine, vinylically unsaturated carboxylic acids having a total of from 3 to 5 carbon atoms, amino-lower alkyl- (the term "amino" also including quaternary ammonium), mono-lower alkylamino-lower alkyl- and di-lower alkylamino-lower alkyl-acrylates and -methacrylates, and allyl alcohol.

29. A polymeric network consisting essentially of a polymer according to claim 24 in crosslinked form.

30. A polymeric network consisting essentially of a polymer according to claim 25 in crosslinked form.

31. A process for the preparation of a polymeric network according to either claim 29 or claim 30, which process comprises crosslinking a polymer according to either claim 24 or claim 25.

32. A moulded article consisting essentially of a polymer according to claim 24.

33. A moulded article consisting essentially of a polymeric network according to either claim 29 or claim 30.

34. A moulded article according to either claim 32 or claim 33 which is a contact lens.

35. A biomedical article consisting essentially of a polymer according to either claim 24 or claim 25.

36. A biomedical article consisting essentially of a polymeric network according to either claim 29 or claim 30.

37. A contact lens consisting essentially of a polymer according to either claim 24 or claim 25.

38. A contact lens consisting essentially of a polymeric network according to either claim 29 or claim 30.

39. The use of a compound of formula (I) according to claim 1 for coating the surface of an article.

40. The use of a polymer according to either claim 24 or claim 25 for coating the surface of an article.

41. The use according to either claim 39 or claim 40, wherein the surface of the article is the surface of a polymer substrate.

42. The use according to either claim 39 or claim 40, wherein the surface of the article is the surface of a contact lens.

43. The use of a polymer according to either claim 24 or claim 25 as a drug delivery system.

44. The use of a polymeric network according to either claim 29 or claim 30 as a drug delivery system.

## Revendications

1. Composé de formule I,
R¹-(COO-alk)ₘ-(OCONH-R)ₙ-(NHCO)ₚ-Y-Z (I)
dans laquelle R¹ représente un groupe hydrocarboné polymérisable par voie radicalaire ; m, n et p ont les valeurs 0 ou 1 ; alk représente un alkylène ayant jusqu'à 10 atomes de carbone ; R représente un diradical ayant jusqu'à 20 atomes de carbone d'un diisocyanate organique ; Z représente un radical monovalent, diminué d'un seul groupe hydroxyle primaire, d'un mono-, di- ou tri-saccharide, d'un oligosaccharide ou d'une cyclodextrine (CD) ; et Y représente -O- ou -NH- ; sous réserve que, lorsque p vaut 0, m et n valent également 0 et Y représente -NH- ; en outre sous réserve que, m, n et p valent 0 lorsque Y représente -NH-.

2. Composé selon la revendication 1, caractérisé en ce que Y représente -O-.

3. Composé selon l'une des revendications 1 ou 2, caractérisé en ce que m = 1, p = 1 et n = 0.

4. Composé selon la revendication 1, caractérisé en ce que m et n valent 0.

5. Composé selon la revendication 1, caractérisé en ce que R¹ représente un alcényle ayant 2 à 12 atomes de carbone.

6. Composé selon la revendication 1, caractérisé en ce que alk représente un alkylène inférieur ayant jusqu'à 7 atomes de carbone.

7. Composé selon la revendication 1, caractérisé en ce que le diradical R représente un alkylène inférieur, un arylène, un groupe cycloaliphatique bivalent saturé ayant 6 à 10 atomes de carbone, un alkylène arylène, un arylène alkylène ou un arylène alkylène arylène.

8. Composé selon la revendication 1, caractérisé en ce que le radical Z dérive d'un monosaccharide qui est choisi parmi l'aldopentose, l'aldohexose, le cétopentose et le cétohexose.

9. Composé selon la revendication 1, caractérisé en ce que le radical Z dérive d'un disaccharide qui est choisi parmi le tréhalose, le maltose, l'isomaltose, le cellobiose, le gentiobiose, le saccharose et le lactose.

10. Composé selon la revendication 1, caractérisé en ce que le radical Z dérive d'un trisaccharide qui est choisi parmi le raffinose, le panose et le maltotriose.

11. Composé selon la revendication 1, caractérisé en ce que le radical Z dérive d'un oligosaccharide qui est choisi parmi le maltotétraose, le maltohexaose et le chitoheptaose.

12. Composé selon la revendication 1, caractérisé en ce que le radical Z dérive d'une cyclodextrine qui est choisie parmi l'α-, la β- et la γ-cyclodextrine.

13. Composé selon la revendication 1, caractérisé en ce que le radical Z dérive d'un monosaccharide qui est choisi parmi l'aldohexose et le cétohexose.

14. Composé selon la revendication 1, caractérisé en ce que le radical Z dérive d'un disaccharide qui est choisi parmi l'α,α-, l'α,β- et le β,β-tréhalose.

15. Composé selon la revendication 1, caractérisé en ce que le radical Z dérive d'une cyclodextrine qui est choisie parmi l'α- et la β-cyclodextrine.

16. Composé selon la revendication 1, caractérisé en ce que le radical Z dérive d'un monosaccharide qui représente un 1,1-alkylglucoside.

17. Composé selon la revendication 1, caractérisé en ce que le radical Z dérive d'un disaccharide qui représente l'α,α-tréhalose.

18. Composé selon la revendication 1, caractérisé en ce que le radical Z dérive d'une cyclodextrine qui représente une α-cyclodextrine.

19. Composé selon la revendication 1, caractérisé en ce que R¹ représente un alcényle ayant 2 à 8 atomes de carbone ; m et p ont la valeur 1 et n vaut 0 ; alk représente un alkylène inférieur ayant jusqu'à 4 atomes de carbone; Y représente -O-; et le radical Z dérive d'un saccharide qui représente un 1-alkylglucoside, un α,α-tréhalose ou une α-cyclodextrine.

20. Composé selon la revendication 1, caractérisé en ce que R¹ représente un alcényle ayant 2 à 8 atomes de carbone ; m, n et p valent 0 ; Y représente -NH- ; et le radical Z dérive d'un saccharide qui représente un 1-alkylglucoside, un α,α-tréhalose ou une α-cyclodextrine.

21. Composé selon la revendication 1, caractérisé en ce que R¹ représente un alcényle ayant 2 à 8 atomes de carbone ; m et n ont la valeur 0 et p vaut 1 ; et le radical Z dérive d'un saccharide qui représente un 1-alkylglucoside, un α,α-tréhalose ou une α-cyclodextrine.

22. Composé selon la revendication 2, caractérisé en ce que R¹ représente un alcényle ayant 2 à 4 atomes de carbone ; et alk représente un alkylène ayant jusqu'à 4 atomes de carbone.

23. Procédé de préparation d'un composé de formule (I) selon la définition de la revendication 1, caractérisé en ce qu'on fait réagir un saccharide de formule (II)
Z-X (II)
dans laquelle Z représente un radical monovalent, diminué d'un seul groupe hydroxyle primaire, d'un mono-, di- ou trisaccharide, d'un oligosaccharide, ou d'une cyclodextrine (CD) ; et X représente un groupe réactif qui est lié au radical monovalent, diminué du groupe hydroxyle primaire, d'un saccharide Z ;
avec un dérivé de formule (III)
R¹-(COO-alk)ₘ-(OCONH-R)ₙ-NCO (III)
ou avec un dérivé de formule (IV)
R¹-NH₂ (IV)
où R¹ représente un groupe hydrocarboné polymérisable par voie radicalaire; m, n et p ont les valeurs 0 ou 1 ; alk représente un alkylène ayant jusqu'à 10 atomes de carbone ; et R représente un diradical ayant jusqu'à 20 atomes de carbone d'un diisocyanate organique.

24. Polymère contenant un produit de polymérisation d'au moins un composé de formule (1) selon la définition de la revendication 1 et, le cas échéant, d'au moins un autre comonomère vinylique (a) qui en diffère, à l'exception d'un polymère que l'on peut obtenir par réaction de 6-iodo-6-désoxy-β-cyclodextrine avec une polyallylamine.

25. Polymère selon la revendication 24, caractérisé en ce que le comonomère (a) est absent et en ce qu'il s'agit d'un homopolymère.

26. Polymère selon la revendication 24, caractérisé en ce que la proportion pondérale par rapport au polymère total d'un composé de formule (I) se situe dans un intervalle de 100 à 0,5 %.

27. Polymère selon la revendication 24, caractérisé en ce que le comonomère (a) est hydrophobe, et est choisi parmi les acrylates et méthacrylates d'alkyle en C₁ à C₁₈ et de cycloalkyle en C₃ à C₁₈, les alkyles en C₃ à C₁₈-acrylamides et méthacrylamides, l'acrylonitrile, le méthacrylonitrile, les vinylalcanoates en C₁ à C₁₈, les alcènes en C₂ à C₁₈, les haloalcènes en C₂ à C₁₈, le styrène, les alkyle inférieur-styrènes, les alkyle inférieur-vinyléthers, les perfluoroalkyles en C₂ à C₁₀-acrylates et -méthacrylates, ou les acrylates et méthacrylates partiellement fluorés correspondants, les perfluoroalkyle en C₃ à C₁₂-éthylthiocarbonylaminoéthyl-acrylates et -méthacrylates, les acryloxy- et méthacryloxy-alkylsiloxanes, le N-vinylcarbazole et les alkylesters en C₁ à C₁₂ de l'acide maléique, de l'acide fumarique, de l'acide itaconique et de l'acide mésaconique.

28. Polymère selon la revendication 24, caractérisé en ce que le comonomère (a) est hydrophile et est choisi parmi les acrylates et méthacrylates d'alkyle inférieur substitués par un hydroxy, l'acrylamide, le méthacrylamide, les alkyle inférieur acrylamides et -méthacrylamides, les acrylates et méthacrylates éthoxylés, les alkyle inférieur acrylamides et méthacrylamides substitués par un hydroxy, les alkyle inférieur vinyléthers substitués par un hydroxy, le vinylsulfonate de sodium, le styrène sulfonate de sodium, l'acide 2-acrylamido-2-méthylpropane sulfonique, le N-vinylpyrrole, la N-vinyl-2-pyrrolidone, la 2- et la 4-vinylpyridine, les acides carboxyliques vinyliquement insaturés ayant au total 3 à 5 atomes de carbone, les aminoalkyle inférieur-(l'expression "amino" comprenant également l'ammonium quaternaire), les mono-alkyle inférieur amino-alkyle inférieur- et dialkyle inférieur amino-alkyle inférieur -acrylates et -méthacrylates et l'alcool allylique.

29. Réseau polymère constitué essentiellement d'un polymère selon la revendication 24 sous forme réticulée.

30. Réseau polymère constitué essentiellement d'un polymère selon la revendication 25 sous forme réticulée.

31. Procédé de préparation d'un réseau polymère selon l'une des revendications 29 ou 30, caractérisé en ce qu'on réticule un polymère selon l'une des revendications 24 ou 25.

32. Article moulé constitué essentiellement d'un polymère selon la revendication 24.

33. Article moulé constitué essentiellement d'un réseau polymère selon l'une des revendications 29 ou 30.

34. Article moulé selon l'une des revendications 32 ou 33, caractérisé en ce qu'il s'agit d'une lentille de contact.

35. Article biomédical constitué essentiellement d'un polymère selon l'une des revendications 24 ou 25.

36. Article biomédical constitué essentiellement d'un réseau polymère selon l'une des revendications 29 ou 30.

37. Lentille de contact constituée essentiellement d'un polymère selon l'une des revendications 24 ou 25.

38. Lentille de contact constituée essentiellement d'un réseau polymère selon l'une des revendications 29 ou 30.

39. Utilisation d'un composé de formule (I) selon la revendication 1 pour revêtir une surface corporelle.

40. Utilisation d'un polymère selon l'une des revendications 24 ou 25 pour revêtir une surface corporelle.

41. Utilisation selon la revendication 39 ou 40, caractérisée en ce que la surface corporelle est une surface de substrat polymère.

42. Utilisation selon la revendication 39 ou 40, caractérisée en ce que la surface corporelle est une surface de lentille de contact.

43. Utilisation d'un polymère selon l'une des revendications 24 ou 25, comme système d'administration d'une substance active pharmaceutique.

44. Utilisation d'un réseau polymère selon l'une des revendications 29 ou 30 comme système d'administration d'une substance active pharmaceutique.
